# EUROPEAN PATENT SPECIFICATION

(11) **EP 3 065 765 B1**
(45) Date of publication and mention of the grant of the patent: **08.04.2020**
(21) Application number: 14860161.0
(22) Date of filing: 06.11.2014
(51) Int. Cl.: A61P 1/18, C07K 14/54, A61K 38/20, A61K 9/00

(54) **USE OF IL-22 DIMERS IN MANUFACTURE OF MEDICAMENTS FOR TREATING PANCREATITIS**
VERWENDUNG VON IL-22-DIMEREN BEI DER HERSTELLUNG VON MEDIKAMENTEN ZUR BEHANDLUNG VON PANKREATITIS
UTILISATION DE DIMÈRES D'IL-22 DANS LA FABRICATION DE MÉDICAMENTS POUR LE TRAITEMENT DE LA PANCRÉATITE

(30) Priority: 07.11.2013 CN 201310549648
(43) Date of publication of application: 14.09.2016
(73) Proprietor: Generon (Shanghai) Corporation Ltd., China (Shanghai) Pilot Free Trade Zone Shanghai 201203 (CN)
(72) Inventor: YAN, Xiaoqiang, Shanghai 201315 (CN); HUANG, Cheng, Shanghai 201315 (CN); WU, Dongdong, Shanghai 201315 (CN); TANG, Kaiyang, Shanghai 201315 (CN); HUANG, Yuliang, Shanghai 201315 (CN)
(74) Representative: Carpmaels & Ransford LLP
(86) International application number: PCT/CN2014/090519
(87) International publication number: WO 2015/067198

(56) References cited:
- WO-A1-2006/073508
- WO-A2-2014/145016
- US-A1- 2013 171 100
- DECHUN FENG ET AL.: 'Interleukin-22 Ameliorates Cerulein-Induced Pancreatitis in Mice by hibiting the Autophagic Pathway' INT. J. BIOL. SCI. 31 December 2012, pages 249 - 257, XP055342720

### SEQUENCE LISTING

<110> Generon (Shanghai) Corporation Ltd.
<120> Use of IL-22 DIMERS IN MANUFACTURE OF A MEDICAMENT FOR TREATING
   PANCREATITIS
<130> P2014-1426
<150> CN201310549648X
   <151> 2013-11-07
<160> 10
<170> Patent In version 3.5
<210> 1
   <211> 16
   <212> PRT
   <213> Artificial sequence
<220>
   <221> MISC_FEATURE
   <223> short peptide
<400> 1
   Gly Ser Gly Gly Gly Ser Gly Gly Gly Gly Ser Gly Gly Gly Gly Ser
   1               5                   10                  15
<210> 2
   <211> 223
   <212> PRT
   <213> Artificial sequence
<220>
   <221> MISC_FEATURE
   <223> IgG2 Fc
<400> 2
   Val Glu Cys Pro Pro Cys Pro Ala Pro Pro Val Ala Gly Pro Ser Val
   1               5                   10                  15
   Phe Leu Phe Pro Pro Lys Pro Lys Asp Thr Leu Met Ile Ser Arg Thr
               20                  25                  30
   Pro Glu Val Thr Cys Val Val Val Asp Val Ser His Glu Asp Pro Glu
           35                  40                  45
   Val Gln Phe Asn Trp Tyr Val Asp Gly Val Glu Val His Asn Ala Lys
       50                  55                  60
   Thr Lys Pro Arg Glu Glu Gln Phe Asn Ser Thr Phe Arg Val Val Ser
   65                  70                  75                  80
   Val Leu Thr Val Val His Gln Asp Trp Leu Asn Gly Lys Glu Tyr Lys
                   85                  90                  95
   Cys Lys Val Ser Asn Lys Gly Leu Pro Ala Ser Ile Glu Lys Thr Ile
               100                 105                 110
   Ser Lys Thr Lys Gly Gln Pro Arg Glu Pro Gln Val Tyr Thr Leu Pro
           115                 120                 125
   Pro Ser Arg Glu Glu Met Thr Lys Asn Gln Val Ser Leu Thr Cys Leu
       130                 135                 140
   Val Lys Gly Phe Tyr Pro Ser Asp Ile Ala Val Glu Trp Glu Ser Asn
   145                 150                 155                 160
   Gly Gln Pro Glu Asn Asn Tyr Lys Thr Thr Pro Pro Met Leu Asp Ser
                   165                 170                 175
   Asp Gly Ser Phe Phe Leu Tyr Ser Lys Leu Thr Val Asp Lys Ser Arg
               180                 185                 190
   Trp Gln Gln Gly Asn Val Phe Ser Cys Ser Val Met His Glu Ala Leu
           195                 200                 205
   His Asn His Tyr Thr Gln Lys Ser Leu Ser Leu Ser Pro Gly Lys
       210                 215                 220
<210> 3
   <211> 146
   <212> PRT
   <213> Artificial sequence
<220>
   <221> MISC_FEATURE
   <223> IL-22
<400> 3
   Ala Pro Ile Ser Ser His Cys Arg Leu Asp Lys Ser Asn Phe Gln Gln
   1               5                   10                  15
   Pro Tyr Ile Thr Asn Arg Thr Phe Met Leu Ala Lys Glu Ala Ser Leu
               20                  25                  30
   Ala Asp Asn Asn Thr Asp Val Arg Leu Ile Gly Glu Lys Leu Phe His
           35                  40                  45
   Gly Val Ser Met Ser Glu Arg Cys Tyr Leu Met Lys Gln Val Leu Asn
       50                  55                  60
   Phe Thr Leu Glu Glu Val Leu Phe Pro Gln Ser Asp Arg Phe Gln Pro
   65                  70                  75                  80
   Tyr Met Gln Glu Val Val Pro Phe Leu Ala Arg Leu Ser Asn Arg Leu
                   85                  90                  95
   Ser Thr Cys His Ile Glu Gly Asp Asp Leu His Ile Gln Arg Asn Val
               100                 105                 110
   Gln Lys Leu Lys Asp Thr Val Lys Lys Leu Gly Glu Ser Gly Glu Ile
           115                 120                 125
   Lys Ala Ile Gly Glu Leu Asp Leu Leu Phe Met Ser Leu Arg Asn Ala
       130                 135                 140
   Cys Ile
   145
<210> 4
   <211> 385
   <212> PRT
   <213> Artificial sequence
<220>
   <221> MISC_FEATURE
   <223> IL-22£-linker£-IgG2 Fc
<400> 4
   Ala Pro Ile Ser Ser His Cys Arg Leu Asp Lys Ser Asn Phe Gln Gln
   1               5                   10                  15
   Pro Tyr Ile Thr Asn Arg Thr Phe Met Leu Ala Lys Glu Ala Ser Leu
               20                  25                  30
   Ala Asp Asn Asn Thr Asp Val Arg Leu Ile Gly Glu Lys Leu Phe His
           35                  40                  45
   Gly Val Ser Met Ser Glu Arg Cys Tyr Leu Met Lys Gln Val Leu Asn
       50                  55                  60
   Phe Thr Leu Glu Glu Val Leu Phe Pro Gln Ser Asp Arg Phe Gln Pro
   65                  70                  75                  80
   Tyr Met Gln Glu Val Val Pro Phe Leu Ala Arg Leu Ser Asn Arg Leu
                   85                  90                  95
   Ser Thr Cys His Ile Glu Gly Asp Asp Leu His Ile Gln Arg Asn Val
               100                 105                 110
   Gln Lys Leu Lys Asp Thr Val Lys Lys Leu Gly Glu Ser Gly Glu Ile
           115                 120                 125
   Lys Ala Ile Gly Glu Leu Asp Leu Leu Phe Met Ser Leu Arg Asn Ala
       130                 135                 140
   Cys Ile Gly Ser Gly Gly Gly Ser Gly Gly Gly Gly Ser Gly Gly Gly
   145                 150                 155                 160
   Gly Ser Val Glu Cys Pro Pro Cys Pro Ala Pro Pro Val Ala Gly Pro
                   165                 170                 175
   Ser Val Phe Leu Phe Pro Pro Lys Pro Lys Asp Thr Leu Met Ile Ser
               180                 185                 190
   Arg Thr Pro Glu Val Thr Cys Val Val Val Asp Val Ser His Glu Asp
           195                 200                 205
   Pro Glu Val Gln Phe Asn Trp Tyr Val Asp Gly Val Glu Val His Asn
       210                 215                 220
   Ala Lys Thr Lys Pro Arg Glu Glu Gln Phe Asn Ser Thr Phe Arg Val
   225                 230                 235                 240
   Val Ser Val Leu Thr Val Val His Gln Asp Trp Leu Asn Gly Lys Glu
                   245                 250                 255
   Tyr Lys Cys Lys Val Ser Asn Lys Gly Leu Pro Ala Ser Ile Glu Lys
               260                 265                 270
   Thr Ile Ser Lys Thr Lys Gly Gln Pro Arg Glu Pro Gln Val Tyr Thr
           275                 280                 285
   Leu Pro Pro Ser Arg Glu Glu Met Thr Lys Asn Gln Val Ser Leu Thr
       290                 295                 300
   Cys Leu Val Lys Gly Phe Tyr Pro Ser Asp Ile Ala Val Glu Trp Glu
   305                 310                 315                 320
   Ser Asn Gly Gln Pro Glu Asn Asn Tyr Lys Thr Thr Pro Pro Met Leu
                   325                 330                 335
   Asp Ser Asp Gly Ser Phe Phe Leu Tyr Ser Lys Leu Thr Val Asp Lys
               340                 345                 350
   Ser Arg Trp Gln Gln Gly Asn Val Phe Ser Cys Ser Val Met His Glu
           355                 360                 365
   Ala Leu His Asn His Tyr Thr Gln Lys Ser Leu Ser Leu Ser Pro Gly
       370                 375                 380
   Lys
   385
<210> 5
   <211> 308
   <212> PRT
   <213> Artificial sequence
<220>
   <221> MISC_FEATURE
   <223> IL-22£-linker£-IL-22
<400> 5
   Ala Pro Ile Ser Ser His Cys Arg Leu Asp Lys Ser Asn Phe Gln Gln
   1               5                   10                  15
   Pro Tyr Ile Thr Asn Arg Thr Phe Met Leu Ala Lys Glu Ala Ser Leu
               20                  25                  30
   Ala Asp Asn Asn Thr Asp Val Arg Leu Ile Gly Glu Lys Leu Phe His
           35                  40                  45
   Gly Val Ser Met Ser Glu Arg Cys Tyr Leu Met Lys Gln Val Leu Asn
       50                  55                  60
   Phe Thr Leu Glu Glu Val Leu Phe Pro Gln Ser Asp Arg Phe Gln Pro
   65                  70                  75                  80
   Tyr Met Gln Glu Val Val Pro Phe Leu Ala Arg Leu Ser Asn Arg Leu
                   85                  90                  95
   Ser Thr Cys His Ile Glu Gly Asp Asp Leu His Ile Gln Arg Asn Val
               100                 105                 110
   Gln Lys Leu Lys Asp Thr Val Lys Lys Leu Gly Glu Ser Gly Glu Ile
           115                 120                 125
   Lys Ala Ile Gly Glu Leu Asp Leu Leu Phe Met Ser Leu Arg Asn Ala
       130                 135                 140
   Cys Ile Gly Ser Gly Gly Gly Ser Gly Gly Gly Gly Ser Gly Gly Gly
   145                 150                 155                 160
   Gly Ser Ala Pro Ile Ser Ser His Cys Arg Leu Asp Lys Ser Asn Phe
   165 170 175
   Gln Gln Pro Tyr Ile Thr Asn Arg Thr Phe Met Leu Ala Lys Glu Ala
   180 185 190
   Ser Leu Ala Asp Asn Asn Thr Asp Val Arg Leu Ile Gly Glu Lys Leu
   195 200 205
   Phe His Gly Val Ser Met Ser Glu Arg Cys Tyr Leu Met Lys Gln Val
   210 215 220
   Leu Asn Phe Thr Leu Glu Glu Val Leu Phe Pro Gln Ser Asp Arg Phe
   225 230 235 240
   Gln Pro Tyr Met Gln Glu Val Val Pro Phe Leu Ala Arg Leu Ser Asn
   245 250 255
   Arg Leu Ser Thr Cys His Ile Glu Gly Asp Asp Leu His Ile Gln Arg
   260 265 270
   Asn Val Gln Lys Leu Lys Asp Thr Val Lys Lys Leu Gly Glu Ser Gly
   275 280 285
   Glu Ile Lys Ala Ile Gly Glu Leu Asp Leu Leu Phe Met Ser Leu Arg
   290 295 300
   Asn Ala Cys Ile
   305
<210> 6
   <211> 375
   <212> PRT
   <213> Artificial sequence
<220>
   <221> MISC_FEATURE
   <223> IL-22-linker-IgG2 Fc
<400> 6
   Ala Pro Ile Ser Ser His Cys Arg Leu Asp Lys Ser Asn Phe Gln Gln
   1 5 10 15
   Pro Tyr Ile Thr Asn Arg Thr Phe Met Leu Ala Lys Glu Ala Ser Leu
   20 25 30
   Ala Asp Asn Asn Thr Asp Val Arg Leu Ile Gly Glu Lys Leu Phe His
   35 40 45
   Gly Val Ser Met Ser Glu Arg Cys Tyr Leu Met Lys Gln Val Leu Asn
       50                  55                  60
   Phe Thr Leu Glu Glu Val Leu Phe Pro Gln Ser Asp Arg Phe Gln Pro
   65                  70                  75                  80
   Tyr Met Gln Glu Val Val Pro Phe Leu Ala Arg Leu Ser Asn Arg Leu
                   85                  90                  95
   Ser Thr Cys His Ile Glu Gly Asp Asp Leu His Ile Gln Arg Asn Val
               100                 105                 110
   Gln Lys Leu Lys Asp Thr Val Lys Lys Leu Gly Glu Ser Gly Glu Ile
           115                 120                 125
   Lys Ala Ile Gly Glu Leu Asp Leu Leu Phe Met Ser Leu Arg Asn Ala
       130                 135                 140
   Cys Ile Ala Ser Thr Lys Gly Pro Val Glu Cys Pro Pro Cys Pro Ala
   145                 150                 155                 160
   Pro Pro Val Ala Gly Pro Ser Val Phe Leu Phe Pro Pro Lys Pro Lys
                   165                 170                 175
   Asp Thr Leu Met Ile Ser Arg Thr Pro Glu Val Thr Cys Val Val Val
               180                 185                 190
   Asp Val Ser His Glu Asp Pro Glu Val Gln Phe Asn Trp Tyr Val Asp
           195                 200                 205
   Gly Val Glu Val His Asn Ala Lys Thr Lys Pro Arg Glu Glu Gln Phe
       210                 215                 220
   Asn Ser Thr Phe Arg Val Val Ser Val Leu Thr Val Val His Gln Asp
   225                 230                 235                 240
   Trp Leu Asn Gly Lys Glu Tyr Lys Cys Lys Val Ser Asn Lys Gly Leu
                   245                 250                 255
   Pro Ala Ser Ile Glu Lys Thr Ile Ser Lys Thr Lys Gly Gln Pro Arg
               260                 265                 270
   Glu Pro Gln Val Tyr Thr Leu Pro Pro Ser Arg Glu Glu Met Thr Lys
           275                 280                 285
   Asn Gln Val Ser Leu Thr Cys Leu Val Lys Gly Phe Tyr Pro Ser Asp
       290                 295                 300
   Ile Ala Val Glu Trp Glu Ser Asn Gly Gln Pro Glu Asn Asn Tyr Lys
   305                 310                 315                 320
   Thr Thr Pro Pro Met Leu Asp Ser Asp Gly Ser Phe Phe Leu Tyr Ser
                   325                 330                 335
   Lys Leu Thr Val Asp Lys Ser Arg Trp Gln Gln Gly Asn Val Phe Ser
               340                 345                 350
   Cys Ser Val Met His Glu Ala Leu His Asn His Tyr Thr Gln Lys Ser
           355                 360                 365
   Leu Ser Leu Ser Pro Gly Lys
       370                 375
<210> 7
   <211> 385
   <212> PRT
   <213> Artificial sequence
<220>
   <221> MISC_FEATURE
   <223> IgG2 Fc-linker-IL-2
<400> 7
<210> 8
   <211> 375
   <212> PRT
   <213> Artificial sequence
<220>
   <221> MISC_FEATURE
   <223> IgG2 Fc-linker-IL-22
<400> 8
   Val Glu Cys Pro Pro Cys Pro Ala Pro Pro Val Ala Gly Pro Ser Val
   1 5 10 15
   Phe Leu Phe Pro Pro Lys Pro Lys Asp Thr Leu Met Ile Ser Arg Thr
   20 25 30
   Pro Glu Val Thr Cys Val Val Val Asp Val Ser His Glu Asp Pro Glu
   35 40 45
   Val Gln Phe Asn Trp Tyr Val Asp Gly Val Glu Val His Asn Ala Lys
   50 55 60
   Thr Lys Pro Arg Glu Glu Gln Phe Asn Ser Thr Phe Arg Val Val Ser
   65 70 75 80
   Val Leu Thr Val Val His Gln Asp Trp Leu Asn Gly Lys Glu Tyr Lys
   85 90 95
   Cys Lys Val Ser Asn Lys Gly Leu Pro Ala Ser Ile Glu Lys Thr Ile
   100 105 110
   Ser Lys Thr Lys Gly Gln Pro Arg Glu Pro Gln Val Tyr Thr Leu Pro
   115 120 125
   Pro Ser Arg Glu Glu Met Thr Lys Asn Gln Val Ser Leu Thr Cys Leu
   130 135 140
   Val Lys Gly Phe Tyr Pro Ser Asp Ile Ala Val Glu Trp Glu Ser Asn
   145 150 155 160
   Gly Gln Pro Glu Asn Asn Tyr Lys Thr Thr Pro Pro Met Leu Asp Ser
   165 170 175
   Asp Gly Ser Phe Phe Leu Tyr Ser Lys Leu Thr Val Asp Lys Ser Arg
               180                 185                 190
   Trp Gln Gln Gly Asn Val Phe Ser Cys Ser Val Met His Glu Ala Leu
           195                 200                 205
   His Asn His Tyr Thr Gln Lys Ser Leu Ser Leu Ser Pro Gly Lys Ala
       210                 215                 220
   Ser Thr Lys Gly Pro Ala Pro Ile Ser Ser His Cys Arg Leu Asp Lys
   225                 230                 235                 240
   Ser Asn Phe Gln Gln Pro Tyr Ile Thr Asn Arg Thr Phe Met Leu Ala
                   245                 250                 255
   Lys Glu Ala Ser Leu Ala Asp Asn Asn Thr Asp Val Arg Leu Ile Gly
               260                 265                 270
   Glu Lys Leu Phe His Gly Val Ser Met Ser Glu Arg Cys Tyr Leu Met
           275                 280                 285
   Lys Gln Val Leu Asn Phe Thr Leu Glu Glu Val Leu Phe Pro Gln Ser
       290                 295                 300
   Asp Arg Phe Gln Pro Tyr Met Gln Glu Val Val Pro Phe Leu Ala Arg
   305                 310                 315                 320
   Leu Ser Asn Arg Leu Ser Thr Cys His Ile Glu Gly Asp Asp Leu His
                   325                 330                 335
   Ile Gln Arg Asn Val Gln Lys Leu Lys Asp Thr Val Lys Lys Leu Gly
               340                 345                 350
   Glu Ser Gly Glu Ile Lys Ala Ile Gly Glu Leu Asp Leu Leu Phe Met
           355                 360                 365
   Ser Leu Arg Asn Ala Cys Ile
       370                 375
<210> 9
   <211> 228
   <212> PRT
   <213> homo sapiens
<400> 9
   Glu Arg Lys Cys Cys Val Glu Cys Pro Pro Cys Pro Ala Pro Pro Val
   1               5                   10                  15
   Ala Gly Pro Ser Val Phe Leu Phe Pro Pro Lys Pro Lys Asp Thr Leu
               20                  25                  30
   Met Ile Ser Arg Thr Pro Glu Val Thr Cys Val Val Val Asp Val Ser
           35                  40                  45
   His Glu Asp Pro Glu Val Gln Phe Asn Trp Tyr Val Asp Gly Val Glu
       50                  55                  60
   Val His Asn Ala Lys Thr Lys Pro Arg Glu Glu Gln Phe Asn Ser Thr
   65                  70                  75                  80
   Phe Arg Val Val Ser Val Leu Thr Val Val His Gln Asp Trp Leu Asn
                   85                  90                  95
   Gly Lys Glu Tyr Lys Cys Lys Val Ser Asn Lys Gly Leu Pro Ala Pro
               100                 105                 110
   Ile Glu Lys Thr Ile Ser Lys Thr Lys Gly Gln Pro Arg Glu Pro Gln
           115                 120                 125
   Val Tyr Thr Leu Pro Pro Ser Arg Glu Glu Met Thr Lys Asn Gln Val
       130                 135                 140
   Ser Leu Thr Cys Leu Val Lys Gly Phe Tyr Pro Ser Asp Ile Ser Val
   145                 150                 155                 160
   Glu Trp Glu Ser Asn Gly Gln Pro Glu Asn Asn Tyr Lys Thr Thr Pro
                   165                 170                 175
   Pro Met Leu Asp Ser Asp Gly Ser Phe Phe Leu Tyr Ser Lys Leu Thr
               180                 185                 190
   Val Asp Lys Ser Arg Trp Gln Gln Gly Asn Val Phe Ser Cys Ser Val
           195                 200                 205
   Met His Glu Ala Leu His Asn His Tyr Thr Gln Lys Ser Leu Ser Leu
       210                 215                 220
   Ser Pro Gly Lys
   225
<210> 10
   <211> 6
   <212> PRT
   <213> Artificial sequence
<220>
   <221> MISC_FEATURE
   <223> short peptide
<400> 10
   Ala Ser Thr Lys Gly Pro
   1               5

## Description

### FIELD OF INVENTION

This invention relates to the area of biological and medical technologies, in particular, this invention relates to the use of interleukin-22 (IL-22) dimer in treating pancreatitis.

### BACKGROUND

It is generally believed that pancreatitis is caused by conversation of trypsinogen to trypsin followed by self-digestion of pancreatic acinar cells. According to the course of disease, pancreatitis can be divided into two forms, acute pancreatitis (AP) and chronic pancreatitis (CP).

The major etiologies of acute pancreatitis are gallstones (including biliary microlithiasis), hypertriglyceridemia and alcohol *etc.* Other causes include overeating, shock, pregnancy, parasitization, hyperlipaemia, hypercalcemia, parotitis, *etc.* Moreover, AP is also associated with iatrogenic factors such as abdominal operation, endoscopic retrograde cholangiopancreatography (ERCP), endoscopic ultrasonography (EUS), *etc*., and medications such as hydrochlorothiazide, ethacrynic acid, indometacin, oral contraceptive, etc. In addition, there are a number of patients with unknown etiology, namely, idiopathic pancreatitis.

Acute pancreatitis is a clinically common disease, the basic lesions of acute pancreatitis include edema, hemorrhage, and necrosis. According to reports, overall, 85% of patients have interstitial pancreatitis, 15% (range 4-47%) have necrotizing pancreatitis. Approximately 10% of patients with interstitial pancreatitis experience organ failure, but in the majority it is transient with a very low mortality. Among patients with necrotizing pancreatitis, 33% (range 16-47%) have infected necrosis, median prevalence of organ failure in necrotizing pancreatitis is 54% (range 29-78%). Prevalence of organ failure is the same or somewhat higher in infected necrosis (34-89%) than in sterile necrosis (45-73%). The overall mortality in acute pancreatitis is approximately 5%: 3% in interstitial pancreatitis, 17% in necrotizing pancreatitis (30% in infected necrosis, 12% in sterile necrosis). The mortality in the absence of organ failure is 0 , with single organ failure is 3% (range 0-8%), with multisystem organ failure 47% (range *28-69%*)(Peter A. Banks et al., Am J Gastroenterol,101: 2379-2400,2006). Currently, the treatment for acute pancreatitis is supportive and consists of fluid and salt replacement and pain management. No specifically effective drugs are available.

Chronic pancreatitis is localized or diffuse pancreatic inflammation caused by various etiological factors. Recurrent severe acute pancreatitis may evolve into chronic pancreatitis (Klöppel G, Maillet B. Hepatogastroenterology, 38(5):408-12, 1991). The mechanisms underlying the pathogenesis of chronic pancreatitis are not fully understood. The current treatments mainly include alternative therapy by the supply of trypsin and treating complications, such as pain management.

Feng et al reported Interleukin-22 ameliorates cerulein-induced pancreatitis in mice (Int. J. Biol. Sci, 8(2), 249-257, 2012). US2013/171100 discusses the use of interleukin-22 in treating viral hepatitis. WO2006073508 describes the use of IL-22 for the treatment of conditions of metabolic disorders. WO2014145016 discusses IL-22 polypeptides and IL-22 Fc fusion proteins and methods of use.

### SUMMARY OF INVENTION

It is an object of the present disclosure to provide the use of IL-22 dimer in the manufacture of a medicament for prevention and/or treatment of pancreatitis.

In one aspect of the present disclosure, a use of interleukin-22 (IL-22) dimer in the manufacture of a medicament for treatment and/or prevention of pancreatitis is provided. The invention provides an IL-22 dimer for use in a method of treating pancreatitis in an individual, wherein the method comprises administering to the individual an effective amount of the IL-22 dimer, wherein the IL-22 dimer is administered at the effective amount of 2 µg/kg to 200 µg/kg.

In some embodiments, the pancreatitis is selected from the group consisting of acute pancreatitis, chronic pancreatitis, alcoholic pancreatitis, recurrent pancreatitis, bile reflux pancreatitis, interstitial pancreatitis, necrotizing pancreatitis, and post ERCP pancreatitis.

In some embodiments, the IL-22 dimer is shown as Formula I:

M1-L-M2 I

wherein,
M1 is a first monomer of IL-22,
M2 is a second monomer of IL-22, and
L is a linker connecting said first monomer and said second monomer and disposed therebetween.

In some embodiments, the IL-22 dimer retains the biological activity of IL-22 and has a serum half-life of longer than twice of that of either the first or the second monomer.

In some embodiments, the serum half-life of the IL-22 dimer is longer than three, five, or ten times of that of the first and/or the second monomer.

In a preferred embodiment, the linker L is selected from the group consisting of:
(i). a short peptide comprising 3 to 50 amino acids; and
(ii). a polypeptide of Formula II:

   -Z-Y-Z- II

   wherein,
   Y is a carrier protein,
   Z is nothing, or a short peptide(s) comprising 1 to 30 amino acids, and
   "-" is a chemical bond or a covalent bond.

In some embodiments, the "-" is a peptide bond.

In some embodiments, Z is 5-50 amino acid residues in length.

In some embodiments, Z comprises the sequence of SEQ ID NO: 1 or SEQ ID NO: 10.

In some embodiments, Z has the sequence of SEQ ID NO: 1 or SEQ ID NO: 10.

In some embodiments, the carrier protein contains at least two cysteines capable of forming intermolecular disulfide bonds.

In some embodiments, the carrier protein is disposed at the N-terminal of IL-22 monomer.

In some embodiments, the carrier protein is disposed at the C-terminal of IL-22 monomer.

In some embodiments, the carrier protein is albumin or Fc fragment of human IgG.

In some embodiments, Fc fragment contains CH2 and CH3 domains.

In some embodiments, Fc fragment comprises the sequence of SEQ ID NO: 2 or SEQ ID NO: 9.

In some embodiments, Fc fragment has the sequence of SEQ ID NO: 2 or SEQ ID NO: 9.

In some embodiments, the IL -22 dimer is formed by two monomeric subunits wherein each monomeric subunit comprises an IL-22 domain, a dimerization domain and optionally a linker connecting the IL-22 domain and the dimerization domain.

In some embodiments, the IL -22 domain is IL-22 monomer, the dimerization domain comprises Fc fragment of human immunoglobulin (such as IgG1, IgG2, IgG3, or IgG4), the optional linker is a peptide connecting the IL-22 monomer and Fc fragment, and the dimer is formed by the connection of two dimerization domains (such as Fc Fragment) via one or more disulfide bond(s).

In some embodiments, the number of said disulfide bond is 2-4.

In some embodiments, the monomeric subunit of each IL-22 dimer comprises an amino acid sequence selected from SEQ ID NO:4 and SEQ ID NOs: 6-8.

In some embodiments, the first monomer and the second monomer of the IL-22 dimer are identical.

In some embodiments, the first monomer and the second monomer are different.

In some embodiments, the biological activity of the IL-22 dimer is selected from one or more biological activities in a group consisting of:
(a) reducing the levels of amylase and/or lipase *in vivo,*
(b) ameliorating pancreatic edema *in vivo,*
(c) inhibiting necrosis of acinar cells and/or adipocytes in pancreas *in vivo,*
(d) ameliorating the infiltration of inflammatory cells in pancreas *in vivo.*

In another aspect of the present disclosure, there is provided a pharmaceutical composition for prevention and/or treatment of pancreatitis, which comprises a pharmaceutically acceptable carrier(s) and a IL-22 dimer of Formula I:

M1-L-M2 I

wherein,
M1 is a first monomer of IL-22;
M2 is a second monomer of IL-22; and
L is a linker connecting said first monomer and said second monomer and disposed therebetween,

In some embodiments, the IL-22 dimer retains the biological activity of IL-22 and has a serum half-life of longer than twice of that of either the first or the second monomer.

In another aspect of the present disclosure, there is provided a use of IL-22 dimer in the manufacture of a medicament for increasing the serum level of amyloid A protein (SAA) and/or C-reactive protein (CRP), and/or reducing the serum level of triglyceride (TG).

In another aspect of the present disclosure, there is provided a method of treating pancreatitis in an individual, comprising administering (such as intravenously administering) to the individual an effective amount of an IL-22 dimer.

In some embodiments, the IL-22 dimer comprises two monomeric subunits, wherein each monomeric subunit comprises an IL-22 domain and a dimerization domain.

In some embodiments, each monomeric subunit of the IL-22 dimer comprises an IL-22 domain linked to a dimerization domain via an optional linker sequence.

In some embodiments, the linker sequence is about 5 to about 50 amino acids.

In some embodiments, the linker sequence comprises the sequence of SEQ ID NO: 1 or SEQ ID NO: 10.

In some embodiments, the linker sequence has the sequence of SEQ ID NO: 1 or SEQ ID NO: 10.

In some embodiments, the dimerization domain comprises at least two cysteines capable of forming intermolecular disulfide bonds.

In some embodiments, the dimerization domain comprises at least a portion of the Fc fragment.

In some embodiments, the Fc fragment comprises CH2 and CH3 domains.

In some embodiments, the Fc fragment comprises the sequence of SEQ ID NO: 2 or SEQ ID NO: 9.

In some embodiments, the Fc fragment has the sequence of SEQ ID NO: 2 or SEQ ID NO: 9.

In some embodiments, the IL-22 domain of each monomeric subunit has the sequence of SEQ ID NO: 3.

In some embodiments, the each monomeric subunit has the sequence selected from SEQ ID NO: 4 and SEQ ID NOs: 6-8.

In some embodiments, the IL-22 dimer is administered intravenously.

In some embodiments, the IL-22 dimer is administered at the amount of about 2 µg/kg to about 200 µg/kg.

In some embodiments, the IL-22 dimer is administered at the amount of about 5 µg/kg to about 80 µg/kg.

In some embodiments, the IL-22 dimer is administered at the amount of about 10 µg/kg to about 45 µg/kg.

In some embodiments, the pancreatitis is acute pancreatitis.

In some embodiments, the pancreatitis is chronic pancreatitis.

In some embodiments, the pancreatitis is interstitial pancreatitis.

In some embodiments, the pancreatitis is necrotizing pancreatitis.

In some embodiments, the pancreatitis is alcohol induced pancreatitis.

In some embodiments, the pancreatitis is recurrent pancreatitis.

In some embodiments, the pancreatitis is bile reflux pancreatitis.

In some embodiments, the pancreatitis is ERCP induced pancreatitis.

In some embodiments, the individual has elevated serum amylase, elevated serum lipase, elevated urine amylase, or elevated C-reactive protein.

In some embodiments, the IL-22 dimer is administered no more than about once every week.

In some embodiments, the IL-22 dimer is administered no more than about once every month.

In some embodiments, the IL-22 dimer is administered no more than about once every three months.

In some embodiments, the effective amount of the IL-22 dimer leads to an increased level of serum amyloid A protein or C-reactive protein in the individual.

In some embodiments, the effective amount of the IL-22 dimer leads to inhibition of triglyceride in the individual.

In some embodiments, the effective amount of the IL-22 dimer does not lead to an elevated level of an inflammatory cytokine.

In some embodiments, the inflammatory cytokine is selected from TNF-α, IL-6, IL-1β, and IL-8.

In some embodiments, the effective amount of the IL-22 dimer leads to a decreased level of amylase and/or lipase *in vivo.*

In some embodiments, the effective amount of the IL-22 dimer ameliorates the pancreatic edema *in vivo.*

In some embodiments, the effective amount of the IL-22 dimer inhibits the necrosis of acinar cells and/or adipocytes in pancreas *in vivo.*

In some embodiments, the effective amount of the IL-22 dimer ameliorates the inflammatory cell infiltration in pancreas *in vivo.*

In some embodiments, the dimerization domain is at the N-terminus of the IL-22 dimer subunit.

In some embodiments, the dimerization domain is at the C-terminus of IL-22 dimer subunit.

### BRIEF DESCRIPTION OF FIGURES

FIG. 1 is an illustration of an exemplary IL-22 dimer according to the present invention. In the figure, "-" represents a linker and the oval-shaped object labeled with "IL-22" represents an IL-22 monomer.

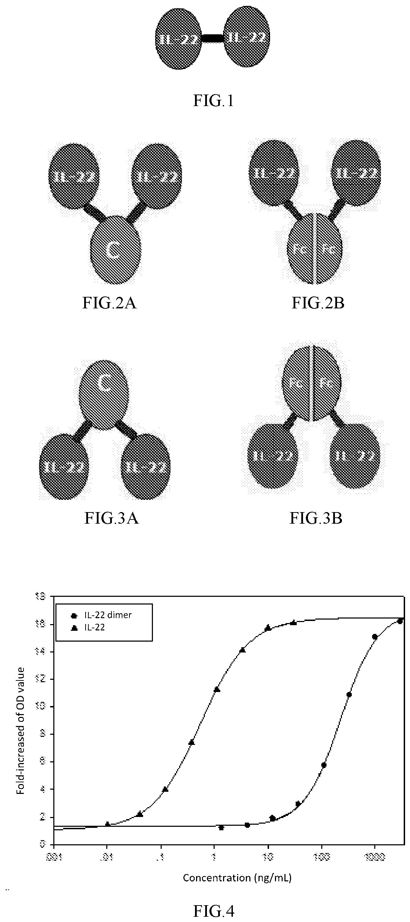

FIGS. 2A and 2B are illustrations of exemplary IL-22 dimers according to the present invention. In the figures, "-" represents an amino acid linker and the oval-shaped object labeled with "IL-22" represents an IL-22 monomer. As illustrated in FIG. 2A, the oval-shaped object labeled with "C" represents a carrier protein wherein the IL-22 is disposed at the N-terminal of the carrier protein. As illustrated in FIG. 2B, the half oval-shaped object labeled with "Fc" represents an Fc fragment which is a dimerization domain, showing a dimer is formed by the coupling of two Fc fragments via disulfide bond(s).
FIGS. 3A and 3B are illustrations of exemplary IL-22 dimers according to the present invention. In the figures, "-" represents an amino acid linker, the oval-shaped object labeled with "IL-22" represents an IL-22 monomer. As illustrated in FIG. 3A, the oval-shaped object labeled with "C" represents a carrier protein wherein the IL-22 is disposed at the C-terminal of the carrier protein. As illustrated in FIG. 3B, the half oval-shaped object labeled with "Fc" represents an Fc fragment which is a dimerization domain, showing a dimer is formed by the coupling of two Fc fragments via disulfide bond(s).
FIG. 4 shows the proliferative effect of IL-22 and IL-22 dimer on Colo205 cells in *in vitro* activity experiment.
FIG. 5 shows the effect of IL-22 and IL-22 dimer on stimulating STAT3 in Colo205 cells in *in vitro* activity experiment.
FIG. 6 shows the distribution of IL-22 dimer in pancreatic tissues in rats after administration. SD rats received a single intravenous injection of 30 µg/kg ¹²⁵I labeled IL-22 dimer via cauda vein. The radioactivity counts in organ tissues were measured at 2, 24, and 48 hrs respectively after the injection.
FIG. 7 shows the distribution of IL-22 dimer in pancreatic tissues in cynomolgus monkeys after administration. Cynomolgus monkeys received a single intravenous injection of 100 µg/kg IL-22 dimer. The drug concentrations in the organ tissues were measured at 2 hrs after the injection.
FIG. 8A shows the changes of the serum levels of amyloid protein (SAA) in human with the time after intravenous administration of IL-22 dimer.
FIG. 8B shows the changes of the serum levels of C-reactive protein in human with the time after intravenous administration of IL-22 dimer.
FIG. 8C shows the changes of the serum levels of triglyceride in human with the time after intravenous administration of IL-22 dimer.
FIG. 8D shows the effect on the serum levels of various cytokines in human with the time after intravenous administration of IL-22 dimer.
FIG. 9A shows the effect of IL-22 and IL-22 dimer on serum amylase levels in pancreatitis model rats.
FIG. 9B shows the effect of IL-22 and IL-22 dimer on serum lipase levels in pancreatitis model rats.

### DETAILED DESCRIPTION OF THE INVENTION

The present application provides an IL-22 dimer for use in a method of treating pancreatitis in an individual by administering (such as intravenously administering) an effective amount of an IL-22 dimer, wherein the IL-22 dimer is administered at the effective amount of 2 µg/kg to 200 µg/kg. Using a well-established animal model for pancreatitis, inventors have demonstrated that IL-22 dimers are highly effective in treating pancreatitis, and can significantly reduce the level of serum amylase and/or lipase in an individual of pancreatitis, ameliorate pancreatic edema in vivo, inhibit necrosis of acinar cells and/or adipocytes in pancreas, and ameliorate the inflammatory cell infiltration in pancreas in vivo. It was surprisingly found that, although IL-22 dimers show decreased activities in vitro assays, at equal molar dose, the in vivo bioactivity of IL-22 dimer is significantly stronger than that of IL-22 monomer. It was further surprisingly found that, while subcutaneous injection of IL-22 dimer results in delayed adverse events at the injection sites, intravenous injection of IL-22 dimer shows excellent tolerability and safety. Furthermore, the administration of IL-22 dimer does not lead to an increased serum level of an inflammatory cytokine in human.

Thus, in some embodiments, there is provided an IL-22 dimer for use in a method of treating pancreatitis, comprising administering (such as intravenously administering) to the individual an effective amount of an IL-22 dimer, wherein the IL-22 dimer is administered at the effective amount of 2 µg/kg to 200 µg/kg. In some embodiments, the IL-22 dimer is administered by intravenous push (IVP). In some embodiments, the IL-22 dimer is administered by intravenous infusion. In some embodiments, the IL-22 dimer is administered by continuous intravenous infusion.

Also disclosed is provided a method of inhibiting inflammation in an individual having pancreatitis, comprising administering (such as intravenously administering) to the individual an effective amount of an IL-22 dimer.

Also disclosed is provided a method of reducing one or more symptoms of pancreatitis in an individual, comprising administering (such as intravenously administering) to the individual an effective amount of an IL-22 dimer.

Also disclosed is provided a method of improving quality of life in an individual having pancreatitis, comprising administering (such as intravenously administering) to the individual an effective amount of an IL-22 dimer.

In some embodiments, the effective amount of the IL-22 dimer leads to a decreased level of serum amylase in an individual having pancreatitis. A method of decreasing level of serum amylase in an individual having pancreatitis, comprising administering (such as intravenously administering) to the individual an effective amount of an IL-22 dimer is disclosed. In some embodiments, the level of serum amylase is decreased at least about any of 2×, 3×, 4×, 5×, 6×, 7×, 8×, 9×, 10x compared to the level prior to the treatment. In some embodiments, the effective amount of the IL-22 dimer leads to a decreased level of serum lipase in an individual having pancreatitis. A method of decreasing level of serum lipase in an individual having pancreatitis, comprising administering (such as intravenously administering) to the individual an effective amount of an IL-22 dimer is disclosed. In some embodiments, the level of serum lipase is decreased at least about any of 2×, 3×, 4×, 5×, 6×, 7×, 8×, 9×, 10× compared to the level prior to the treatment. In some embodiments, the effective amount of IL-22 dimer inhibits necrosis of acinar cells and/or adipocytes in pancreas, ameliorate edema and inflammatory cell infiltration in pancreas in an individual having pancreatitis. A method of inhibiting necrosis of acinar cells and/or adipocytes in the pancreas of an individual having pancreatitis, comprising administering (such as intravenously administering) to the individual an effective amount of an IL-22 dimer is disclosed. A method of ameliorating edema and inflammatory cell in filtration in the pancreas of an individual having pancreatitis, comprising administering (such as intravenously administering) to the individual an effective amount of an IL-22 dimer is disclosed.

A method of preventing and/or treating pancreatitis, comprising administering (such as intravenously administering) to an individual an effective amount of an IL-22 dimer, wherein the effective amount of the IL-22 dimer leads to an increased level of serum amyloid A protein in the individual is disclosed. A method of increasing level of serum amyloid A protein in an individual having pancreatitis, comprising administering (such as intravenously administering) to the individual an effective amount of an IL-22 dimer is disclosed. In some embodiments, the level of serum amyloid A protein is increased at least about any of 5×, 10×, 15×, 20×, 25×, 30×, 50×, 100×, 150×, 200×, 500×, 1000×, 2000×, or 2500× compared to the level prior to the treatment. A method of preventing and/or treating pancreatitis, comprising administering (such as intravenously administering) to an individual an effective amount of an IL-22 dimer, wherein the effective amount of the IL-22 dimer leads to an increased level of C- reactive protein in the individual is disclosed. A method of increasing level of C-reactive protein in an individual having pancreatitis, comprising administering (such as intravenously administering) to the individual an effective amount of an IL-22 dimer is disclosed. In some embodiments, the level of C-reactive protein is increased at least about any of 2×, 3×, 4×, 5×, 10×, 15×, 20×, 25×, 30×, 50×, or 100× compared to the level prior to the treatment. A method of preventing and/or treating pancreatitis, comprising administering (such as intravenously administering) to an individual an effective amount of an IL-22 dimer, wherein the effective amount of the IL-22 dimer leads to a decreased level of triglyceride in the individual is disclosed. A method of decreasing the level of triglyceride in an individual having pancreatitis, comprising administering (such as intravenously administering) to the individual an effective amount of an IL-22 dimer. In some embodiments, the level of triglyceride is decreased at least about any of 2×, 3×, 4×, 5×, 10×, 15×, 20×, 25×, or 30× compared to the level prior to the treatment is disclosed.

A method of treating pancreatitis, comprising administering (such as intravenously administering) to an individual an effective amount of an IL-22 dimer, wherein the effective amount of the IL-22 dimer does not lead to an elevated level of an inflammatory cytokine (such as any one of more inflammatory cytokines selected from the group consisting of TNFα, IL-6, IL-1β, and IL-8) is disclosed. A method of treating pancreatitis, comprising administering (such as intravenously administering) to an individual an effective amount of an IL-22 dimer, wherein the effective amount of the IL-22 dimer leads to two or more of the aforesaid treatment endpoints, including, but not limited to, decreased level of serum amylase, decreased level of serum lipase, inhibition of necrosis of acinar cells and/or adipocyte in pancreas, ameliorated edema and inflammatory cell infiltration in pancreas, increased level of serum amyloid A protein, increased level of C-reactive protein, and decreased level of triglyceride is disclosed.

As used in the invention and disclosure, the term "therapy" refers to administration of IL-22 dimer to a subject in need thereof in order to cure, ameliorate, improve, reduce, delay, and/or affect the disease, symptom, or predisposition of the subject.

The term "effective dose" refers to a dose of IL-22 dimer which can achieve the goal of treatment within the subject in need thereof. It is to be understood by one of ordinary skills in the art that, "therapeutically effective dose" may vary depending on the routes of administration, the types of excipients used and the combination with other medicaments. The term "the individual to be treated" or "individual" refers to a mammal, such as humans. An individual includes, but is not limited to, human, bovine, horse, feline, canine, rodent, or primate. In some embodiments, the individual is human. In some embodiments, the individual to be treated is 16 years of age or younger, 18 years of age or younger, 25 years of age or younger, 35 years of age or younger, 45 years of age or younger, 55 years of age or younger, 65 years of age or younger, or 75 years of age or younger. In some embodiments, individual to be treated is 16 years of age or older, 18 years of age or older, 25 years of age or older, 35 years of age or older, 45 years of age or older, 55 years of age or older, 65 years of age or older, or 75 years of age or older.

In some embodiments, the individual to be treated has an Ranson criteria score of 1 or more, of 2 or more, of 3 or more, of 4 or more, of 5 or more, of 6 or more, of 8 or more, of 9 or more, of 10 or more, or of 11 or more. In some embodiments, the individual to be treated has a CT Severity Index score of 1 or more, of 2 or more, of 3 or more, or of 4. In some embodiments, the individual has a Modified CT Severity Index score of 1 or more, of 2 or more, of 3 or more, of 4 or more, of 5 or more, of 6 or more, of 7 or more, of 8 or more, of 9 or more, or of 10. In some embodiments, the individual has an APACHE II score of 1 or more, of 2 or more, of 3 or more, of 4 or more, 5 or more, of 6 or more, of 7 or more, of 8 or more, of 9 or more, of 10 or more, of 11 or more, of 15 or more, of 20 or more, of 25 or more, of 30 or more, of 35 or more, or of 40 or more. In some embodiments, the individual has an APACHE III score of 1 or more, of 2 or more, of 3 or more, of 4 or more, 5 or more, of 10 or more, of 15 or more, of 20 or more, of 25 or more, of 30 or more, of 35 or more, of 40 or more, of 45 or more, or of 50 or more. In some embodiments, the individual to be treated has a Modified Glasgow score of 1 or more, of 2 or more, of 3 or more, of 4 or more, of 5 or more, of 6 or more, of 7 or more, or of 8 or more. In some embodiments, the individual has two or more of the characteristics described above. In some embodiments, the individual to be treated is characterized by the typical imaging changes of acute pancreatitis. In some embodiments, the individual to be treated exhibits one or more of symptoms of pancreatitis, including, but not limited to, nausea, abdominal pain or tenderness, vomiting, indigestion, weight loss, steatorrhea, pathological change, acute inflammation, vacuolization (such as acinar vacuolization), neutrophil infiltration, edema, pancreatic stones, gallstones, biliary stones, pancreatic cysts, elevated or depressed body temperature, elevated heart rate, elevated respiratory rate, internal hemorrhaging, jaundice, diabetes, necrosis (such as acinar necrosis), pancreatic cancer, and organ failure.

In some embodiments, the individual to be treated has an elevated serum amylase levels (such as greater than about 120 U/L, greater than about 150 U/L, greater than about 200 U/L, greater than about 300 U/L, or greater than about 400 U/L), elevated urine amylase (such as greater than about 40 U/h, greater than about 80 U/h, greater than about 120 U/h, or greater than about 160 U/h). In some embodiments, the individual to be treated has an elevated serum lipase levels (such as greater than about 150 U/L, greater than about 200 U/L, greater than about 300 U/L, or greater than about 400 U/L, or greater than about 500 U/L). In some embodiments, the individual to be treated has a serum amylase level and/or a serum lipase level which is/are at least 3 times of that of the upper limit of normal value. In some embodiments, the individual to be treated has an elevated serum C-reactive protein (such as greater than about 25 mg/L, greater than about 50 mg/L, greater than about 100 mg/L, greater than about 150 mg/L, greater than about 200 mg/L, greater than about 250 mg/L, greater than about 300 mg/L, or greater than about 400 mg/L).

In some embodiments, the individual to be treated has an elevated serum alanine transaminase (such as greater than about 40 U/L, greater than about 50 U/L, greater than about 60 U/L, greater than about 75 U/L, or greater than about 150 U/L). In some embodiments, the individual to be treated has an elevated serum IL-6 or IL-8 levels or elevated blood phospholipase A₂ (such as greater than about 3 U/L, greater than about 7 U/L, greater than about 15 U/L, greater than about 30 U/L, greater than about 60 U/L, or greater than about 100 U/L). In some embodiments, the individual to be treated has an elevated serum procalcitonin level (such as greater than about 3 ng/mL, greater than about 4 ng/mL, greater than about 5 ng/mL, or greater than about 7.5 ng/mL). In some embodiments, the individual to be treated has an elevated urinary trypsinogen activation peptide (such as greater than about 3 ng/mL, greater than about 6 ng/mL, greater than about 10 ng/mL, greater than about 20 ng/mL, or greater than about 30 ng/mL). In some embodiments, the individual to be treated has an elevated serum trypsinogen activation peptide. In some embodiments, the individual to be treated has a decreased level of stool pancreatic elastase (such as less than about 200 µg/g, less than about 150 µg/g, less than about 100 µg/g, or less than about 50 µg/g). In some embodiments, the individual to be treated has an elevated white blood cell count (such as greater than about 12000 cells/mm³, greater than about 14000 cells/mm³, greater than about 16000 cells/mm³, greater than about 18000 cells/mm³, or greater than about 20000 cells/mm³). In some embodiments, the individual to be treated has a decreased serum calcium level (such as less than about 10 mg/mL, less than about 8 mg/mL, less than about 6 mg/mL, less than about 4 mg/mL, or less than about 2 mg/mL). In some embodiments, the individual to be treated has an elevated serum aspartate transaminase (such as greater than about 30 U/L, greater than about 40 U/L, greater than about 50 U/L, greater than about 60 U/L, or greater than about 75 U/L). In some embodiments, the individual to be treated has an elevated serum lactate dehydrogenase (such as greater than about 250 IU/L, greater than about 300 IU/L, greater than about 350 IU/L, greater than about 400 IU/L, or greater than about 450 IU/L). In some embodiments, the individual to be treated has an elevated blood glucose (such as greater than about 180 mg/dL, greater than about 200 mg/dL, greater than about 220 mg/dL, greater than about 260 mg/dL, or greater than about 300 mg/dL). In some embodiments, the individual to be treated has a base deficit (such as greater than about 3 mEq/L, greater than about 4 mEq/L, greater than about 5 mEq/L, or greater than about 6 mEq/L).

As used herein, various types of pancreatitis can be prevented or treated.

In some embodiments, the pancreatitis is chronic pancreatitis, which include, but are not limited to, alcoholic pancreatitis (e.g., chronic alcohol use), hereditary pancreatitis, cystic fibrosis pancreatitis, trauma induced pancreatitis, tropical pancreatitis, malnutrition induced pancreatitis, hypercalcemia pancreatitis, calcific pancreatitis (e.g., caused by calcified stones such as pancreatic stones, gallstones, or biliary stones), autoimmune pancreatitis, or idiopathic pancreatitis.

In some embodiments, the pancreatitis is acute pancreatitis. In some embodiments, the acute pancreatitis is an isolated incident. In some embodiments, the acute pancreatitis is recurrent. Acute pancreatitis includes, but is not limited to, alcohol induced pancreatitis (e.g., acute alcohol use), gallstone pancreatitis, biliary pancreatitis, post-endoscopic retrograde cholangiopancreatography pancreatitis, or idiopathic pancreatitis. In some embodiments, the acute pancreatitis is caused by viral infection, such as *coxsackievirus*, *cytomegalovirus*, *hepatitis B*, *herpes simplex virus*, *mumps*, and *varicella-zoster viruses.* In some embodiments, the acute pancreatitis is caused by bacterial infections, such as infections caused by *Legionella*, *Leptospira*, *Mycoplasma*, or *Salmonella.* In some embodiments, the acute pancreatitis results from *Aspergillus* fungal infections. In some embodiments, the acute pancreatitis is caused by parasitic infections, such as *Ascaris*, *Cryptosporidium*, and *Toxoplasma.*

In some embodiments, the pancreatitis results from drug use, such as those used to treat various medical conditions or illicit use. For example, in some embodiments, pancreatitis can be treated when associated with the administration of didanosine, asparaginase, mesalamine, hormones, opiates, tetracycline, cytarabine, ACE inhibitors, valproic acid, azathioprine, sulfasalazine, furosemide, sulindac, steroids, or HMG-CoA reductase inhibitors.

In some embodiments, hereditary pancreatitis or familial pancreatitis may be treated. For example, in some embodiments, pancreatitis in individuals with variations in trypsin-1 (also called cationic trypsinogen or PRSS1), serine protease inhibitor Kazal-type 1 (SPINK1), or cystic fibrosis transmembrane conductance regulator (CFTR) can be treated. In some embodiments, variations are caused by truncations of a responsible gene or coded protein.

In some embodiments, the pancreatitis is associated with other factors, including, but not limited to, hypercalcemia, hyperlipidemia, hyperamylasemia, abdominal trauma, gastric ulcers, scorpion stings, hypothermia, pancreas divisum, pregnancy, diabetes, surgery, pancreatic cancer, and vasculitis. In some embodiments, pancreatitis resulting from autoimmune disease can be treated. In some embodiments, idiopathic pancreatitis may be treated.

In some embodiments the pancreatitis is caused by gallstones or other blockage of the bile ducts. Because the gallbladder and pancreas share a single drainage duct connecting the organs to the gastrointestinal tract, gallstones that lodge in the duct can block the flow of digestive enzymes exiting the pancreas, resulting in acute gallstone pancreatitis. Other forms of biliary pancreatitis may result from passage of biliary sludge or anatomical variations within the bile ducts resulting in biliopancreatic reflux.

In some embodiments, the pancreatitis results from endoscopic retrograde cholangiopancreatography (ERCP). ERCP is often used to diagnose or treat problems or abnormalities within the biliary or pancreatic ductal systems, for example, gallstones, jaundice, or tumors. The procedure, however, occasionally results in pancreatitis.

Pancreatitis can be diagnosed and treatment can be assessed with various methods, which include, but are not limited to, ultrasound (such as endoscopic ultrasound), computed tomography (CT), magnetic resonance imaging (MRI), or other internal visualization technologies. Endoscopic retrograde cholangiopancreatography (ERCP) or magnetic resonance cholangiopancreatography (MRCP) may also be used to visualize the pancreas, gallbladder, and bile ducts to diagnose or assess the treatment endpoint for pancreatitis.

For diagnosis and treatment assessment using endoscopic ultrasound, evaluative criteria may include one or more of hyperechoic foci, hyperechoic strands, or hyperechoic lobules on the pancreatic parenchyma, pancreatic cysts, irregular pancreatic duct contour, or dilated pancreatic ducts. CT Severity Index, such as described in Balthazar, et al., Radiology 156:767-772 (1985), Balthazar, Radiology 174:331-336 (1990), or Mortele et al., Am. J. Roentgenology, 183: 1261-1265 (2004), may also be used. In some embodiments, findings of abdominal wall edema or elevated scoring on a MRI Severity Index (MRSI) such as described in Yang, et al., Eur. J. Radiology, 81:3041-3047 (2012) may be used as an evaluative criteria. In some embodiments, visualization of biliary or gallbladder stones may be used as an evaluative criteria.

### IL-22

As used herein, the term "Interleukin-22" or "IL-22" refers to a protein, which (a) has essentially the same amino acid sequence as the human/murine IL-22 as described by Dumoutier et al. in U.S. Pat. No. 6,359,117 and (b) the same biological activity as natural IL-22. IL-22 of the present invention includes but is not limited to human IL-22, recombinant human IL-22, murine IL-22 and/or recombinant murine IL-22.

Specifically, Interleukin-22 (IL-22), also known as IL-10 related T cell-derived inducible factor (IL-TIF), is a glycoprotein expressed in and secreted from activated T cells and natural killer cells (NK cells). Activated T cells are mainly CD4+ cells, especially CD28 pathway activated Tₕ1 cells, Tₕ17 cells and Th22 cells, among others. The expression of IL-22 mRNA was originally identified in IL-9 simulated T cells and mast cells in murine, as well as Concanavilin A (Con A) stimulated spleen cells (Dumoutier, et al., J. Immunology, 164:1814-1819, 2000). The human IL- 22 mRNA is mainly expressed in peripheral T cells upon stimulation by anti-CD3 or Con A.

Native IL- 22 precursor peptide consists of 179 amino acid residues, while the mature peptide consists of 146 amino acid residues. Dumoutier first reported the IL-22 cloned DNA sequences of mouse and human (Dumoutier, et al., 2000; US Pat. No. 6,359,117 and US Pat. No. 6,274,710). IL-22 is mainly expressed in activated T cells(especially Th17 cells), the lectin-stimulated spleen cells (Duroutier JI 2002), IL-2/IL-12-stimulated NK cells (Wolk, K et al, J. Immunology, 168:5379-5402, 2002), and in a number of organs and tissues, including gut, liver, stomach, kidney, lung, heart, thymus, spleen, upon LPS stimulation, in which an increase of the expression of IL-22 in those organs and tissues can be measured. IL-22 expresses its biological function through the combination of IL-22R1 receptor and IL-10R2 receptor. IL-22R1 is a receptor specific to IL-22 and is expressed in skin, kidney, the digestive system (pancreas, small intestine, liver, large intestine, colon), and the respiratory system (lung, bronchi). Published researches demonstrated that IL-22 is an immuno-modulator.

### IL-22 dimer

The structure of the IL-22 dimer of the present invention is exemplified as Formula I. FIGS. 1-3 illustrate the representative structures of the IL-22 dimer of the present invention, in which the carrier protein includes but is not limited to Fc fragment of human IgG (such as IgG1, IgG2, IgG3 or IgG4), or human albumin.

In some embodiments, the IL-22 dimer of the present invention comprises two monomeric subunits, in which each monomeric subunit comprises an IL-22 domain and a dimerization domain. Each monomeric subunit comprises an IL-22 domain linked to a dimerization domain via an optional linker sequence. The IL-22 domain can be at the C terminus or N terminus of the dimerization domain. The carrier protein of the IL-22 dimer is formed by two dimerization domains via dimerization. An amino acid sequence of an exemplary IL-22 dimer is shown in SEQ ID NO: 5 in which amino acid residues 1-146 represent IL-22, amino acid residues 147-162 represent the linker, and residues 163-308 represent another IL-22.

An amino acid sequence of an exemplary IL-22 monomer with Fc fragment, which is used to form the IL-22 dimer of this embodiment, is shown in SEQ ID NO: 4 in which amino acid residues 1-146 represent an IL-22, amino acid residues 147-162 represent the linker, and residues 163-385 represent Fc fragment of human IgG2. A dimer is formed by the two IL-22 monomers with Fc fragment via the coupling of the Fc fragments.

An amino acid sequence of an exemplary IL-22 monomer with Fc fragment, which is used to form the IL-22 dimer of this embodiment, is shown in SEQ ID NO: 6 in which amino acid residues 1-146 represent an IL-22, amino acid residues 147-152 represent the linker, and residues 153-375 represent Fc fragment of human IgG2. A dimer is formed by the two IL-22 monomers with Fc fragment via the coupling of the Fc fragments. An amino acid sequence of an IL-22 monomer with Fc fragment, which is used to form the IL-22 dimer of this embodiment, is shown in SEQ ID NO: 7 in which amino residues 1-223 represent Fc fragment of human IgG2, amino residues 224-239 represent the linker, and residues 240-385 represent an IL-22. A dimer is formed by the two IL-22 monomers with Fc fragment via the coupling of the Fc fragments. An amino acid sequence of an IL-22 monomer with Fc fragment, which is used to form the IL-22 dimer of this embodiment, is shown in SEQ ID NO: 8 in which amino acid residues 1-223 represent Fc fragment of human IgG2, amino acid residues 224-229 represent the linker, and residues 230-375 represent an IL-22. A dimer is formed by the two IL-22 monomers with Fc fragment via the coupling of the Fc fragments.

As used herein and in the claims, the term "linker peptide" or "linker" refers to oligo peptide disposed between one IL-22 monomer and carrier protein, or one IL-22 monomer (or IL-22 domain) and a dimerization domain and connecting the two domains together. There is no special restriction on the length of the linker. A linker is usually 5-50 amino acid residues in length. In general, a linker does not affect or significantly affect the proper fold and conformation formed by the configuration of the two IL-22 monomers. Some examples of linkers include (but are not limited to):

Preferably, the linker contains an amino acid sequence selected from:
(a). an amino acid sequence with 3-16 hydrophobic amino acid residues Gly or Pro, such as Gly-Pro-Gly-Pro-Gly-Pro;
(b). an amino acid sequence encoded by multiple cloning sites. Such sequences usually contain 5-20 amino acid residues, preferably, 10-20 amino acid residues;
(c). an amino acid sequence of a protein other than IL-22 monomer, such as an amino acid sequence of IgG or albumin; and
(d). an amino acid sequence comprising any combination of (a), (b), and (c) above.

In one preferred embodiment, the linker has the sequence of GSGGGSGGGGSGGGGS (i.e. amino acid residues of SEQ ID NO: 1) and ASTKGP (i.e. amino acid residues of SEQ ID NO: 10).

In addition, an amino acid sequence not affecting the biological activity of IL-22 monomer can be added to the N-terminal or C-terminal of the fusion protein. In a preferred embodiment, such appended amino acid sequence is beneficial to expression (e.g. signal peptide), purification (e.g. 6 × His sequence, the cleavage site of Saccharomyces cerevisiae α-factor signal peptide (Glu-Lys-Arg), or enhancement of biological activity of the fusion protein.

In some embodiments, the IL-22 dimer comprises two monomeric subunits, wherein each monomeric subunit comprises an IL-22 domain and a dimerization domain. In some embodiments, the IL-22 domain is fused to the N-terminus of the dimerization domain. In some embodiments, the IL-22 domain is fused to the C-terminus of the dimerization domain. In some embodiments, the IL-22 domain and the dimerization domain are linked via an optional peptide linker (for example a peptide linker of about 5 to about 50 amino acids in length, for example a linker having the sequence of SEQ ID NO:10). In some embodiments, the dimerization domain of IL-22 dimer comprises leucine zippers.

In some embodiments, the IL-22 dimer comprises two IL-22 monomeric subunits, wherein each monomeric subunit comprises an IL-22 monomer and at least a portion of an immunoglobulin Fc fragment ("the Fc fragment", or namely Fc region). In some embodiments, the IL-22 domain is fused to the N-terminus of the Fc fragment. In some embodiments, the IL-22 domain is fused to the C-terminus of the Fc fragment. In some embodiments, the IL-22 domain and the Fc fragment are linked via an optional peptide linker (for example a peptide linker of about 5 to about 50 amino acids in length, for example a linker having the sequence of SEQ ID NO: 1 or SEQ ID NO: 10). In some embodiments, the IL-22 domain has the sequence of SEQ ID NO:3. In some embodiments, the Fc fragment comprises at least two cysteines capable of forming intermolecular disulfide bonds. In some embodiments, the Fc fragment is truncated at the N-terminus, e.g., lacks the first 1, 2, 3, 4, 5, 6, 7, 8, 9, or 10 amino acids of a complete immunoglobulin Fc domain. In some embodiments, the Fc fragment is of type IgG2. In some embodiments, the Fc fragment is of type IgG4. In some embodiments, the Fc fragment has the sequence of SEQ ID NO:2 or SEQ ID NO: 9.

In some embodiments, the IL-22 dimer comprises two IL-22 monomeric subunits, wherein each monomeric subunit comprises (for example has) the sequence of any of SEQ ID NO:4 or SEQ ID NOs: 6-8.

The invention encompasses modifications to the polypeptides described herein, including functionally equivalent proteins which do not significantly affect their properties and variants which have enhanced or decreased activity. Modification of polypeptides is routine practice in the art and need not be described in detail herein. Examples of modified polypeptides include polypeptides with conservative substitutions of amino acid residues, one or more deletions or additions of amino acids which do not significantly deleteriously change the functional activity, non-conservative mutations which do not significantly deleteriously change the functional activity, or use of chemical analogs.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an N-terminal methionyl residue or an epitope tag. Other insertional variants of the IL-22 monomeric subunits include the fusion to the N- or C-terminus of the polypeptide, or a polypeptide which increases the serum half-life of the IL-22 dimer.

Twenty amino acids are commonly found in proteins. Those amino acids can be grouped into nine classes or groups based on the chemical properties of their side chains. Substitution of one amino acid residue for another within the same class or group is referred to herein as a "conservative" substitution. Conservative amino acid substitutions can frequently be made in a protein without significantly altering the conformation or function of the protein. In contrast, non-conservative amino acid substitutions tend to disrupt conformation and function of a protein. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). (See Table 1 below.)

**Table 1: Example of amino acid classification**

| | |
|---|---|
| Small/Aliphatic residues: | Gly, Ala, Val, Leu, Ile |
| Cyclic Imino Acid: | Pro |
| Hydroxyl-containing Residues: | Ser, Thr |
| Acidic Residues: | Asp, Glu |
| Amide Residues: | Asn, Gln |
| Basic Residues: | Lys, Arg |
| Imidazole Residue: | His |
| Aromatic Residues: | Phe, Tyr, Trp |
| Sulfur-containing Residues: | Met, Cys |

In some embodiments, the conservative amino acid substitution comprises substituting any of glycine (G), alanine (A), isoleucine (I), valine (V), and leucine (L) for any other of these aliphatic amino acids; serine (S) for threonine (T) and vice versa; aspartic acid (D) for glutamic acid (E) and vice versa; glutamine (Q) for asparagine (N) and vice versa; lysine (K) for arginine (R) and vice versa; phenylalanine (F), tyrosine (Y) and tryptophan (W) for any other of these aromatic amino acids; and methionine (M) for cysteine (C) and vice versa. Other substitutions can also be considered conservative, depending on the environment of the particular amino acid and its role in the three-dimensional structure of the protein. For example, glycine (G) and alanine (A) can frequently be interchangeable, as can alanine (A) and valine (V). Methionine (M), which is relatively hydrophobic, can frequently be interchanged with leucine and isoleucine, and sometimes with valine. Lysine (K) and arginine (R) are frequently interchangeable in locations in which the significant feature of the amino acid residue is its charge and the differing pKs of these two amino acid residues are not significant. Still other changes can be considered "conservative" in particular environments (see, e.g., Biochemistry at pp. 13-15, 2nd ed. Lubert Stryer ed. (Stanford University); Henikoff et al., Proc. Nat'l Acad. Sci. USA (1992) 89:10915-10919; Lei et al., J. Biol. Chem. (1995) 270(20): 11882-11886).

It was surprising found that although certain IL-22 dimers have less activities than IL-22 in *in vitro* assays, they are significantly more active in an *in vivo* context in treating pancreatitis. For example, in some embodiments, the IL-22 dimer described herein has an EC50 of no less than about 20 ng/mL (including for example no less than about any of 100 ng/mL , 200 ng/mL, 300 ng/mL, 400 ng/mL, or more) in an *in vitro* cell proliferation assay. In some embodiments, the IL-22 dimer has an EC50 that is at least about 5× (including for example at least about 10×, 30×, 50×, 100×, 150×, 300×, 400×, 500×, 600×, 1000× or more) that of a wildtype monomeric IL-22 (for example the monomeric IL-22 having the sequence of SEQ ID NO:3) in an *in vitro* cell proliferation assay. In some embodiments, the IL-22 dimer has an EC50 of no less than about 10 ng/mL (including for example no less than about any of 50 ng/mL, 100 ng/mL, 200 ng/mL, 300 ng/mL, 400 ng/mL, or more) in an *in vitro* STAT3 stimulation assay. In some embodiments, the IL-22 dimer has an EC50 that is at least about 10x (including for example at least about 50×, 100×, 200×, 300×, 400×, 500×, 600×, 700×, 800×, 900×, 1000×, 1500×, or more) that of a wildtype monomeric IL-22 (for example the monomeric IL-22 having the sequence of SEQ ID NO:3) in an *in vitro* STAT3 stimulation assay.

In some embodiments, the IL-22 dimer has a serum half-life that is significantly longer than that of IL-22 monomer. In some embodiments, the IL-22 dimer as a serum half-life of at least about any of 15, 30, 50, 100, 150, 200, 250, 300, or 350 hours. In some embodiments, while the dose of IL-22 dimer is 2 µg/kg, the serum half-life is at least about any of 15, 30, 50, 100, 150, or 200 hours. In some embodiments, while the dose of IL-22 dimer is 10 µg/kg, the serum half-life is at least about any of 50, 100, 150, or 200 hours. In some embodiments, while the dose of IL-22 dimer is 30 µg/kg, the serum half-life is at least about any of 100, 150, 200, or 250 hours. In some embodiments, while the dose of IL-22 dimer is 45 µg/kg, the serum half-life is at least about any of 100, 150, 200, 250, 300, or 350 hours.

### Preparation of IL-22 Dimers

The IL-22 monomeric subunits of the IL-22 dimers may be expressed using recombinant DNA technology. The nucleotide sequence encoding IL-22 monomeric subunits can be inserted into a replicable cloning or protein expression vector at restriction sites using known techniques. In some embodiments, a single nucleotide sequence encoding IL-22 monomeric subunits is inserted into a cloning or expression vector. In some embodiments, a nucleotide sequence encoding the IL-22 region and a nucleotide sequence encoding the extension peptide region may be separately inserted into a cloning or expression vector in such a manner that when the nucleotide sequence is expressed as a protein, a continuous polypeptide is formed. In some embodiments, a nucleotide sequence encoding a linker, a nucleotide sequence encoding a dimerization domain, and a nucleotide sequence encoding an IL-22 region may be separately inserted into a cloning or expression vector in such a manner that when the nucleotide sequence is expressed as a protein, a continuous polypeptide is formed. In some embodiments, the nucleotide sequence encoding IL-22 monomeric subunit may be fused to a nucleotide sequence encoding an affinity or identification tag, such as, but not limited to, a His- tag, FLAG-tag, SUMO-tag, GST-tag, antibody-tag, or MBP-tag. In some embodiments, the cloning or expression vector may be then transfected or transformed into eukaryotic or prokaryotic cells using known techniques. In some embodiments, IL-22 or IL-22 monomeric subunits may be expressed *in vitro.*

The expression host cell may be any cell able to express IL-22 dimers. Suitable prokaryotic expression host cells may include, but are not limited to, *Escherichia coli*, *Erwinia*, *Klebsiella*, *Proteus*, *Salmonella*, S*erratia*, *Shigella*, *Bacillus subtilis*, *Bacillus licheniformis*, *Pseudomonas*, and *Streptomyces.* Eukaryotic cell, such as fungi or yeast, may also be suitable for expression of IL-22 monomeric subunits, for example, but not limited to, *Saccharomyces, Schizosaccharomyces pombe*, *Kluyveromyces lactis*, *Kluyveromyces fragilis*, *Kluyveromyces waltii*, *Kluyveromyces drosophilarum*, *Kluyveromyces thermotolerans*, *Kluyveromyces marxianus*, *Pichia pastoris*, *Neurospora crassa*, *Schwanniomyces*, *Penicillium*, *Tolypocladium, Synechococcus* and *Aspergillus.* Plant or algal cells may also be suitable for expression of IL-22 monomeric subunits, such as *Chlamydomonas.* Eukaryotic cell derived from multicellular organisms may also be suitable for expression of IL-22 monomeric subunits, for example, but not limited to, invertebrate cells such as *Drosophila* S2 and *Spodoptera* Sf9, or mammalian cells such as Chinese Hamster Ovary (CHO) cells, COS cells, human embryonic kidney cells (such as HEK293 cells), murine testis trophoblastic cells, human lung cells, and murine breast cancer cells. After the IL-22 monomeric subunit cloning plasmid is transformed or transfected into a host cell, the host cells can be grown on conventional nutrient media and protein expression induced, if necessary. In some embodiments, the expression of IL-22 monomeric subunits do not require inducement.

In some embodiments, expressed IL-22 monomeric subunits will form IL-22 dimers. In some embodiments, IL-22 monomeric subunits will require further inducement, such as by supplying an oxidation compound (such as hydrogen peroxide or a catalytic metal), UV light, or a chemical crosslinker (such as formaldehyde, 1,6-bismaleimidohexane, 1,3-dibromo-2-propanol, bis(2-chloroethyl)sulfide, or glutaraldehyde).

In some embodiments, the forming of IL-22 dimers do not require inducement. In some embodiments, host cell used to express IL-22 dimers is China Hamster Ovary(CHO cell).In some embodiments, IL-22 dimers may be purified using any number of protein purification techniques. For example, IL-22 dimers may be purified using affinity chromatography, ion exchange chromatography, reverse-phase HPLC, size-exclusion chromatography, precipitation, or ultracentrifugation. In some embodiments, an affinity tag fused to the IL-22 monomeric subunit polypeptide may be removed.

The preparation methods of IL-22 dimers can be referred to the patent application PCT/CN2011/079124 (WO2012/028089) filed by Generon (Shanghai) Corporation, LTD on Aug 30^{th} ,2011.

### Administration of IL-22 dimers

The IL-22 dimers described herein can be administered to an individual via various routes. In some embodiments, the IL-22 dimer can be administered parenterally, intravenously, orally, intramuscularly, or subcutaneously.

In some embodiments, the individual is a mammal, preferably including any of human, rodents, or primates.

The dose of the inventive composition of the IL-22 dimers administered to an individual (such as human) will vary with the particular composition, the method of administration, and the particular type of pancreatitis being treated. The dose should be sufficient to effect a desirable response, such as a therapeutic or prophylactic response against a particular disease. In some embodiments, an effective amount is an amount sufficient to delay the development of the disease. In some embodiments, an effective amount is an amount sufficient to prevent occurrence and/or recurrence. An effective amount can be administered in one or more administrations. A disclosed dosage of the IL-22 dimer includes, for example, about 1.5µg/kg to about 500 µg/kg. According to the invention, the IL-22 dimer is administered at the effective amount of, 2 µg/kg to 200 µg/kg, including for example about 5 µg/kg to about 80 µg/kg, about 10 µg/kg to about 45 µg/kg, or about 30µg/kg to about 40 µg/kg.

In some embodiments, the IL-22 dimer is administered once every week. In some embodiments, the IL-22 dimer is administered once every 2, 3, 4, 5, 6, 7, 8, 9, 10, 11, 12, or 24 weeks. In some embodiments, the IL-22 dimer is administered once every 1, 2, 3, 4, 5, 6, 7, 8, 9, 10, or 12 months. In some embodiments, the IL-22 dimer is administered only once.

Also disclosed is administration of the IL-22 dimer intravenously at the dose of at least about any of 10 µg/kg, 20 µg/kg, 30 µg/kg, 40 µg/kg, or 50 µg/kg. In some embodiments, the IL-22 dimer is administered no more frequent than once every week, once every month, once every two months, or once every six months.

### Kits and Medicines

Also provided are kits, medicines, unit dosage, or products suitable for any one of the methods described herein. For example, in some embodiments, there is provided a kit comprising an IL-22 dimer and an instruction for using the IL-22 dimer for treating pancreatitis.

The IL-22 dimers described herein can be formulated with pharmaceutically acceptable excipients or carriers for use in treating pancreatitis.

The pharmaceutical composition of the present disclosure comprises a safe and effective amount of said IL-22 dimer, or a pharmaceutically acceptable salt, or a pharmaceutically acceptable excipient or carrier. "Safe and effective amount" refers to an amount of a compound sufficient to substantially improve the condition of the patient in need thereof without causing serious side-effects. The safe and effective amount is determined based on the specific circumstances such as age, condition, and regimen associated with a subject of treatment.

"Pharmaceutically acceptable excipient or carrier" refers to one or more compatible solid or liquid filling or gelatin materials which are suitable to be used in human with sufficient purity and sufficiently low toxicity. "Compatibility" refers to the ability of each ingredient of the composition to mutually blend with the compound of the present disclosure and the mutual blending ability between the ingredients, without substantially decreasing the clinical efficacy of the compound. Some of the examples of pharmaceutically acceptable excipient or carrier include cellulose and its derivatives (e.g. sodium carboxymethylcellulose, sodium ethylcellulose, cellulose acetate, *etc.*), gelatin, speckstone, solid lubricating agent (e.g. stearic acid, magnesium stearate), calcium sulphate, plant oil (e.g. pea oil, sesame oil, peanut oil, olive oil, etc.), polyols (e.g. propylene glycol, glycerol, mannitol, sorbitol, etc.), emulsifier (e.g. Tween®), wetting agent (e.g. sodium lauryl sulfate), colorant, flavoring agent, stabilizer, antioxidant, antiseptic, pyrogen-free water, etc.

Route of administration of the IL-22 dimer of the present invention comprises oral administration, rectal administration, parenteral administration (intravenous, intramuscular, or subcutaneous), and local administration.

Solid form for oral administration comprises capsules, tablets, pills, powder, and granules. In these solid forms, active compound is mixed with at least one of the conventionally inert excipients (or carriers), such as sodium citrate, dicalcium phosphate, or any of the following ingredients: (a) filing or bulking agent, e.g. starch, lactose, sucrose, glucose, mannitol, and silicic acid; (b) adhesion agent, e.g. carboxymethylcellulose, alginate, gelatin, polyvinyl pyrrolidone, sucrose, and acacia; (c) humectants, e.g. glycerol; (d) disintegrating agent, e.g. agar, calcium carbonate, potato starch or cassava starch, alginic acid, compounded silicate, and sodium carbonate; (e) buffering agent, e.g. paraffin wax; (f) absorption accelerating agent, e.g. quaternary amine compound; (g) wetting agent, e.g. cetanol and glycerin monostearate; (h) absorbent, e.g. bolus alba; and (i). lubricating agent, e.g. speckstone, calcium stearate, magnesium stearate, solid polyethylene glycol, sodium lauryl sulfate, or any mixture thereof. Capsules, tablets, and pills can also comprise buffering agent.

Solid forms such as tablets, sugar pill, capsules, pills, and granules can be prepared with coating and core-shell materials, such as casing and other materials known in the art. These materials can comprise opacifying agent and the active compound or compound in such composition can be released in a delayed fashion that the release is done in certain part of the alimentary canal. Embedding component such as polymer materials and wax materials can be used. If desired, active compounds can be mixed with one or more of the above-described excipients to formulate a micro capsule form.

Liquid forms for oral administration comprise pharmaceutically acceptable emulsion, solution, suspension, syrup, or tincture. Apart from active compounds, liquid forms can also comprise inert diluents conventionally used in the art such as water or other solvent, solubilizing agent and emulsifier such as ethanol, isopropanol, carbonate acetate, ethyl acetate, propan-2-ol,1,3-butan-2-ol, dimethylformamide, and oil, in particular cotton oil, peanut oil, maize embryo oil, olive oil, castor oil, and sesame oil or any mixture thereof.

Apart from these inert diluents, the composition can also comprise additives, such as wetting agent, emulsifying agent, suspending agent, sweetening agent, correctives, and spices.

Apart from active compounds, suspension can also comprise suspending agent, such as ethoxyl isostearic alcohol, polyoxyethylene sorbitol, sorbitan, microcrystalline cellulose, aluminium methoxide, agar, or any mixture thereof.

Compositions used for parenteral administration can also comprise physiologically acceptable liquid combinations such as sterile water or anhydrous solution, dispersion solution, suspension, or emulsion, etc. Appropriate hydrated or anhydrous carriers, diluting agent, solvent, or excipient comprise water, ethanol, polyols (such as propylene glycol, polyethylene glycol, glycerinum, etc.), and appropriate mixtures thereof, are described in Chinese Pharmacopeia or the other countries' Pharmacopeias. Preferably, the liquid compositions can also comprise pharmaceutically acceptable additives commonly used, provided that the additives should not inhibit the functions of IL-22 dimers. The representative additives include (but are not limited to): buffer, PH adjuster, and the like.

Compositions used for parenteral administration can also comprise sterilized powder (for example, lyophilized powder) that can be reconstituted to an injectable solution or dispersal solution. Preferably, the lyophilized powder can also comprise pharmaceutically acceptable additives commonly used, provided that the additives should not inhibit the functions of IL-22 dimers. The representative additives include (but are not limited to): buffer, PH adjuster. Preferably, the solvents used to dissolve the lyophilized powder include (but are not limited to) glucose solution, sodium chloride solution.

In some embodiments, the IL-22 dimer described herein can be administered intravenously, for example, intravenous push or intravenous infusion.

Forms of the IL-22 dimer of the present invention used for partial administration comprise ointment, powder, patch, sprayer, and inhalant. Under sterile conditions, active components can be mixed with physiologically acceptable carrier and any antiseptic, buffering agent, or propellant if desired.

The IL-22 dimer of the present invention can be solely administered or be administered in conjunction with any other pharmaceutically acceptable compounds.

The micro-capsule containing IL-22 dimer of the present invention can be used as a sustained release system. Sustained release micro-capsule system of recombinant protein has been successfully applied to recombinant human growth hormone (rhGH), recombinant human interferon (rhIFN), IL-2 and MNrgp120 (Johnson et al., Nat. Med., 2:795-799 (1996); Yasuda, Biomed. Ther 27:1221-1223 (1993); WO 97/03692, WO 96/40072, WO 96/07399; US5,654,010).

The sustained release system of IL-22 dimer of the present invention can be prepared with poly(lactic-co-glycolic acid) (PLGA) which has good biologically compatibility and broad biological degradability. Lactic acid and glycolic acid, the degrading products of PLGA, can be cleared quickly in human body. Furthermore, the degradability of that polymer can vary from several months to several years depending on its molecular weight and composition (Lewis, "Controlled release of bioactive agents form lactide/glycolide polymer," in: M. Chasin and R. Langer (Eds.), Biodegradable Polymers as Drug Delivery Systems (Marcel Dekker: New York, 1990), pp. 1-41)).

The dosage and concentration of the pharmaceutical composition of the present invention can be adjusted according to actual use situation. One skilled in the art should know how to choose the suitable dosage and route of administration according to practical needs. The principle for adjusting between different species such as mice and human can be seen in Mordenti, J. and Chappell, W. "The use of interspecies scaling in toxicokinetics" In Toxicokinetics and New Drug Development, Yacobi et al.; Pergamon Press, New York 1989, pp. 42-96.

Also disclosed are articles of manufacture comprising the compositions described herein in suitable packaging. Suitable packaging for compositions described herein are known in the art, and include, for example, vials (such as sealed vials), vessels (such as sealed vessels), ampules, bottles, jars, flexible packaging (e.g., sealed Mylar or plastic bags), and the like. These articles of manufacture may further be sterilized and/or sealed. Also provided are unit dosage forms comprising the compositions described herein. These unit dosage forms can be stored in a suitable packaging in single or multiple unit dosages and may also be further sterilized and sealed.

The present disclosure also provides kits comprising compositions (or unit dosages forms and/or articles of manufacture) described herein and may further comprise instruction(s) on methods of using the composition, such as uses further described herein. In some embodiments, the kit of the disclosure comprises the packaging described above. In other embodiments, the kit of the disclosure comprises the packaging described above. The kits described herein may further include other materials desirable from a commercial and user standpoint, including other buffers, diluents, filters, needles, syringes, and package inserts with instructions for performing any methods described herein.

The main advantages of the present invention include, but are not limited to:
1. Compared to IL-22 monomer, the bioactivity of IL-22 dimer is significantly stronger than that of IL-22 monomer *in vivo.*
2. IL-22 dimer has been proven to be effective in preventing and/or treating pancreatitis in animal model. IL -22 dimer can significantly reduce the level of serum amylase and/or lipase in an individual having pancreatitis, ameliorate pancreatic edema *in vivo,* inhibit necrosis of acinar cells and/or adipocytes and ameliorate the inflammatory cell infiltration in pancreas *in vivo.* At equal molar IL-22 dose, IL-22 dimer shows a better therapeutic efficacy in animal model of pancreatitis compared to IL-22 monomer.
3. It was surprisingly found for the first time that, intravenous injection of IL-22 dimer shows excellent tolerability and safety, whereas subcutaneous injection of IL-22 dimer results in delayed adverse events at the injection sites.
4. IL-22 dimer has a significant biological activity in human, significantly increasing the serum levels of CRP, SAA and reducing the level of serum TG.
5. The administration of IL-22 dimer does not lead to an increased serum level of an inflammatory cytokine in human. It is understood that aspect and embodiments of the invention described herein include "consisting" and/or "consisting essentially of' aspects and embodiments.

Reference to "about" a value or parameter herein includes (and describes) variations that are directed to that value or parameter per se. For example, description referring to "about X" includes description of "X".

As used herein and in the appended claims, the singular forms "a," "or," and "the" include plural referents unless the context clearly dictates otherwise. It is understood that aspects and variations of the invention described herein include "consisting" and/or "consisting essentially of" aspects and variations.

The following exemplary embodiments further describe the present invention. Further, for the embodiments in which details of the experimental methods are not described, such methods are carried out according to conventional conditions such as those described in Sambrook et al. Molecular Cloning: A Laboratory Manual (New York: Cold Spring Harbor Laboratory Press,1989), or as suggested by the manufacturers.

### EXAMPLES

### Example 1 Proliferation effect of IL-22 or IL-22 dimer on Colo205 cells

Colo205 cells were cultured in RPMI1640 10% FBS medium and the cells were grown to the logarithmic phase. Supernatant was discarded and PBS was added to wash away residual culture medium, followed by addition of 2-5 mL 0.25% Trypsin-EDTA for digestion. Then medium was added and mixed to uniformity by pipetting. Mixture was centrifuged at 1500 rpm for 5 min and cells were collected and prepared into 5.0×10⁵ Cell/ml cell suspension with basic medium. The suspension was added into the wells of 96-well plate (100 µL/well) and stayed overnight at 37°C, in 5% CO₂ incubator. On the next day, the 96-well plate was removed from the CO₂ incubator and centrifuged at 800 rpm for 5 minutes at 4°C. Then, 90 µL of cell supernatant was withdrawn from each well and 90 µL 0.1% BSA/RPMI 1640 was added to each well, followed by addition of IL-22 dimer (consisting of two monomeric subunits each comprising a sequence shown in SEQ ID NO: 4) to the final concentration of 1.4, 4.1, 12.3, 37.0, 111.1, 333.3, 1000, 3000 ng/mL, IL-22 (rhIL-22, namely, recombinant human IL-22) to the final concentration of 0.01, 0.04, 0.12, 0.37, 1.1, 3.3, 10, 30 ng/mL. The mixture was incubated for 20 hours at 37°C in 5% CO₂ incubator and cell supernatant was collected and the OD value thereof was tested using IL-10 ELISA kit (R&D, Cat: S1000B).

As shown in FIG. 4, the half effective concentration (EC50) value of IL-22 dimer is 229 ng/mL (2,675 pM) and that of IL-22 is 0.54 ng/mL (32.4 pM). It shows that the bioactivity of IL-22 dimer is far lower than that of IL-22 monomer in *in vitro* activity experiment.

### Example 2 Effect of IL-22 or IL-22 dimer on STAT3 activation in Colo205 cells

Colo205 cells were cultured in RPMI1640 10% FBS medium and the cells were grown to the logarithmic phase. Supernatant was discarded and PBS was added to wash away residual culture medium, followed by addition of 2-5 mL 0.25% Trypsin-EDTA for digestion. Then medium was added and mixed to uniformity by pipetting. Mixture was centrifuged at 1500 rpm for 5 min and cells were collected and prepared into 2.0×10⁵ Cell/ml cell suspension with basic medium RPMI1640. The suspension was added into the wells of 96-well plate (100 µL/well) and stayed at 37°C for 6 hrs, in 5% CO₂ incubator. The suspension was treated respectively with various concentrations of rhIL-22 or IL-22 dimer (consisting of two monomeric subunits each comprising a sequence shown in SEQ ID NO: 4) for 1 hr. After discarding the supernatant, add 40 µL cell lysis buffer (Cat No.9803S, Cell Signaling) into each well. The supernatant was collected by centrifugation. Protein concentration was determined using Bradford method. Additionally, STAT3 phosphorylation level was measured using an ELISA method (STAT3 [pY705] phosphor ELISA kit (Invitrogen, Cat:KH00481). The pSTAT3 content is calculated by dividing the detected concentration of pSTAT3 by protein concentration.

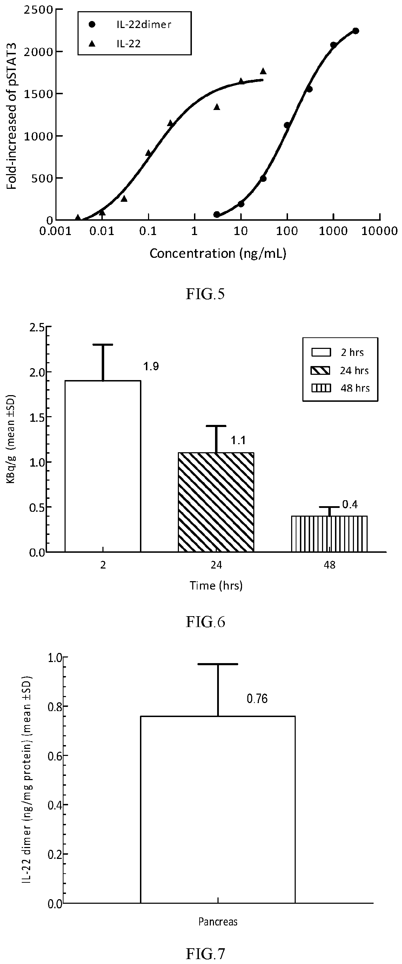

As shown in FIG. 5, the half effective concentration (EC50) value of IL-22 dimer activating STAT3 is 119.5 ng/mL (1394 pM, calculated using the theoretical molecular weight of IL-22 dimer which is 85.7 KD) and that of IL-22 is 0.14 ng/mL (6.9 pM, calculated using the molecular weight of IL-22 which is 16.7KD).

### Example 3 Distribution of IL-22 dimer in organ tissues in SD rats

18 SD rats were randomly divided into 3 groups with 6 animals per group(half male and half female). The animals received a tail vein injection of ¹²⁵I-IL-22 dimer labeled by Iodogen method (consisting of two monomeric subunits each comprising a sequence shown in SEQ ID NO: 4) at a dose of 30 µg/kg. The animals were sacrificed at 2, 24 and 48 hrs after the injection, respectively. The organ tissues were collected and weighed, and the radioactivity counts were measured directly. Then the radioactivity counts per gram of tissues were calculated.

The results showed that the IL-22 dimer was stable in pancreas for 48 hrs after the injection. As shown in FIG.6, the concentrations of IL-22 dimer in pancreas at 24, 48 hrs were decreased to 56% and 21% of that of IL-22 dimer at 2 hrs after the injection, respectively. The concentrations of IL-22 dimer in livers at 24 hrs and 48 hrs were decreased to 28% and 9% of that of IL-22 dimer at 2 hrs after the injection, respectively. At 2 hrs after the injection, the concentrations of IL-22 dimer in pancreas were about 1/5 of that of IL-22 dimer in liver.

### Example 4 Distribution of IL-22 dimer in organ tissues in cynomolgus monkey

3 male cynomolgus monkeys, weighing 4.3-4.6 kg, received intravenous injection of IL-22 dimer (consisting of two monomeric subunits each comprising a sequence shown in SEQ ID NO: 4) at a dose of 100 µg/kg. The animals were sacrificed at 2 hrs after the injection. The organ tissues were collected and stored in liquid nitrogen. The tissues were weighed and lysed by adding the lysis buffer to obtain the tissue homogenate. After centrifugation, the supernatant was separated and subjected to protein concentration determination. The concentrations of IL-22 dimer in the tissues were measured using an ELISA method (Human IL-22 ELISA Kit, Biolegend, Cat.No434506).

The results showed that the concentration of IL-22 dimer in the pancreas was fairly low (about 0.76 ng/mg protein). As shown in FIG.7, this concentration was far lower than that of IL-22 dimer in liver (about 1/5 of the concentration in liver).

### Example 5 Clinical safety of IL-22 dimer in healthy human subject

### Methods:

Healthy male volunteers were enrolled and randomized into 6 dose groups :
Placebo group (n=8): received a single dose of equal volume of 5% glucose/saline via intravenous infusion.

IL-22 dimer 2.0 µg/kg SC dose group (n=6) (SC group): received a single subcutaneous dose of IL-22 dimer at 2.0 µg/kg.

IL-22 dimer 2.0 µg/kg IV dose group (n=6) (IV group): IL-22 dimer were dissolved in 100 mL 5% glucose/saline solution and administered at a single dose of 2 µg/kg via intravenous infusion
IL-22 dimer 10 µg/kg IV dose group (n=6) (IV group): IL-22 dimer were dissolved in 100 mL 5% glucose/saline solution and administered at a single dose of 10 µg/kg via intravenous infusion.

IL-22 dimer 30 µg/kg IV dose group(n=6) (IV group): IL-22 dimer were dissolved in 100 mL 5% glucose/saline solution and administered at a single dose of 30 µg/kg via intravenous infusion.

IL-22 dimer 45 µg/kg IV dose group(n=6) (IV group): IL-22 dimer were dissolved in 100 mL 5% glucose/saline solution and administered at a single dose of 45 µg/kg via intravenous infusion.

Wherein, the IL-22 dimer consisted of two monomeric subunits each comprising a sequence shown in SEQ ID NO: 4.

The safety was evaluated through physical examination, laboratory test, body weight, vital signs, electrocardiogram, and abdomen ultrasound, etc. In addition, the serum level of drug concentration, SAA-1, CRP, TG and cytokines were assayed.

### Results:

### A. adverse events

IL-22 dimer 2.0 µg/kg SC dose group: totally six adverse events considered related to the investigated drug, including injection site dry skin(× 3), berythema(× 2), and nummular eczema(× 1).

IL-22 dimer 2.0 µg/kg IV dose group: no adverse events were observed.

IL-22 dimer 10 µg/kg IV dose group: two adverse events were observed, including chills (an infusion related reaction) (× 1) and headache (× 1).

IL-22 dimer 30 µg/kg IV dose group: six adverse events were observed, including local dry skin(× 4), allergic dermatitis (× 1), and infusion related reaction (× 1).

IL-22 dimer 45 µg/kg IV dose group: twelve adverse events were observed, including local dry skin (6), eye pruritus (× 3), erythematous rash (× 2), and somnolence (× 1).

Placebo group: adverse events including upper respiratory tract infection (× 1), lethargy(× 1) and hyperhidrosis (× 1) were observed.

The results of adverse events, physical examination, laboratory test, body weight, vital signs, electrocardiogram, and abdomen ultrasound data, etc., showed that a single intravenous administration of IL-22 dimer at a dose as high as 45 µg/kg demonstrated a good safety profile with no observed serious adverse events or life-threatening adverse events. Fewer adverse events were reported following IL-22 dimer dosing via IV compared to SC at the 2.0 µg/kg dose level, indicating that IV was much better tolerated by the study subjects (Table 2). The results demonstrated that intravenous administration of IL-22 dimer has a better safety and tolerability compared to subcutaneous administration.

**Table 2 Adverse events at injection site and skin after IL-22 dimer administration**

| Dosing group | Injection site | skin |
|---|---|---|
| placebo | Not observed | Not observed |
| 2 µg/kg, SC | dry skin(×3) ,erythema(×2), and nummular eczema(×1) were observed 10-17 days after the administration | Not observed |
| 2 µg/kg, IV | Not observed | Not observed |
| 10 µg/kg, IV | Not observed | Not observed |
| 30 µg/kg, IV | Not observed | Local dry skin(×4), allergic dermatitis(× 1) |
| 45 µg/kg, IV | Not observed | Local dry skin(×6), eye pruritus (×3), erythematous rash (×2) |

### B. pharmacokinetics of IL-22 dimer in human

The vein blood samples were taken prior to the administration and at different time points following the administration. After centrifugation, the serum was separated and stored at <70 °C. The drug concentration in the serum was measured using an ELISA method (Human IL-22 ELISA Kit, Biolegend, Cat.No 434506). Pharmacokinetic parameters were analyzed using a non-compartmental model on the detected results (analysis software: Phoenix™ WinNonlin® (Pharsight Corporation, Version 6.2.1).The results showed IL-22 dimer had a very excellent half-life in human, among which, the single dose of 45 µg/kg group had a half-life of 206 hrs which was significantly better than that of IL-22 monomer.

**Table 3 Pharmacokinetic parameters (mean value, n=6)**

| Dosage (µg/kg,IV) | Tₘₐₓ (hrs) | Cₘₐₓ (ng/mL) | Tₗₐₛₜ (hrs) | Cₗₐₛₜ (ng/mL) | AUC₀₋ₜ (hr^{∗}ng/mL) | AUC_{0-∞} (hr^{∗}ng/mL) | AUC₀₋₂₄ₕ (hr^{∗}ng/mL) | T_{1/2} (hrs) | Cl (mL/hr/kg) | Vz (mL/kg) |
|---|---|---|---|---|---|---|---|---|---|---|
| 2 | 0.7 | 15.5 | 60 | 3.75 | 437 | 650 | 247 | 39.4 | 3.35 | 177 |
| 10 | 0.2 | 62.3 | 284 | 4.41 | 4150 | 4840 | 1050 | 108 | 2.15 | 330 |
| 30 | 0.2 | 176 | 528 | 6.12 | 15400 | 16900 | 3230 | 161 | 1.82 | 419 |
| 45 | 0.2 | 247 | 528 | 7.73 | 18000 | 20400 | 4340 | 206 | 2.26 | 654 |

C. IL-22 dimer can significantly increase the serum levels of SAA, CRP and decrease serum levels of TG

### a. serum amyloid protein (SAA)

The concentration of serum SAA-1 was measured using an ELISA method (human SAA ELISA kit, Cat No.KHA0011C, Invitrogen).

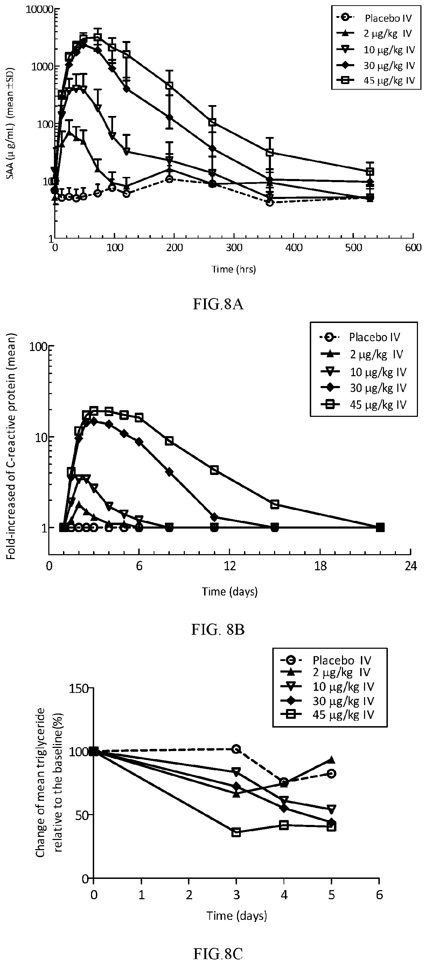

The results showed the IV administration of IL-22 dimer can significantly increase the human serum concentration of SAA, indicating a very significant biological activity. As shown in FIG.8A, compared to the placebo group, the concentration of SAA-1 was significantly increased at 12 hrs after the IL-22 dimer administration. High serum concentration of SAA remain fairly high in the 45 µg/kg dose group on day 15 after the administration.

**Table 4 the maximum concentration (Cmax) and fold-increased of SAA-1**

| Group (IV) (µg/kg) | SAA-1 Cmax | Fold-increased of Cmax (relative to placebo group) |
|---|---|---|
| Placebo | 6* | 1 |
| IL-22 dimer 2 µg/kg ,IV | 71 | 12 |
| IL-22 dimer 10 µg/kg ,IV | 402 | 67 |
| IL-22 dimer 30 µg/kg ,IV | 2355 | 393 |
| IL-22 dimer 45 µg/kg ,IV | 3194 | 532 |

| | | |
|---|---|---|
| * indicating average value of placebo group | | |

### b. C-reactive protein

The levels of C-reactive protein (CRP) were measured using immunity transmission turbidity.

As shown in FIG. 8B, the IV administration of IL-22 dimer significantly increased the serum concentration of C-reactive protein compared to the placebo group.

### c. triglyceride

The changes of serum triglycerides prior to and post the administration were detected using automatic blood biochemistry analyzer.

As shown in FIG.8C, the IV administration of IL-22 dimer significantly reduced the serum levels of triglyceride, exhibiting an obvious dose response relationship compared to the placebo group,.

### d. cytokine assay

The serum samples of placebo group and IL-22 dimer 45 µg/kg IV group were collected before the administration and at 24, 48 hrs after the administration, and were measured using Proteome Profiler Arrays- Human Cytokine Array Panel A (Cat.No.ARY005,

R&D systems) to obtain the levels of various cytokines. The PBMCs (human Peripheral Blood Mononuclear Cells) were treated with 50 ng/mL PMA (phorbol myristate acetate) for 24 hrs and then the supernatant was used as a positive control. 200 µL of each serum samples was loaded and measured following the kit's instruction.

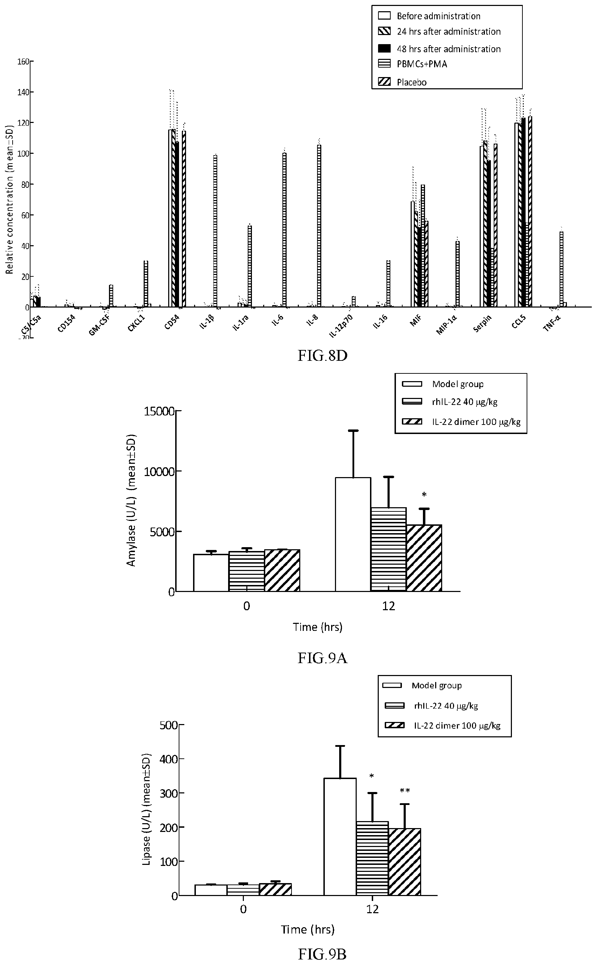

As shown in FIG.8D, the levels of inflammatory cytokines such as TNFα, IL-6, IL-1β, IL-8, etc. were markedly increased in the positive control (PBMCs+PMA). Showing a similar profile to the placebo group, the levels of CD54, MIF, Serpin E1 and CCL5 were relatively higher for the serum samples taken at 24 and 48 hrs after the administration in the IL-22 dimer 45 µg/kg IV group, and the levels of inflammatory cytokines such as TNFα, IL-6, IL-1β, IL-8 did not markedly change compared to that of serum samples taken prior to the administration. These demonstrated that the administration of IL-22 dimer does not lead to increased levels of serum inflammatory cytokines.

### Example 6 Preventive and therapeutic efficacy of IL-22 or IL-22 dimer in rat model of acute pancreatitis induced by retrograde injection of sodium taurocholate into the biliopancreatic duct.

Acute pancreatitis model induced by retrograde injection of sodium taurocholate into the biliopancreatic duct, has been widely used to assess the pathogenesis of bile reflux pancreatitis and the efficacy of a medicament. In this experiment, the rat model of acute pancreatitis was produced by retrograde injection of 0.1 mL/100g 3.5% sodium taurocholate into the biliopancreatic duct.

### SD rats were randomly divided into 3 groups:

Model control group (n=6), received a single intravenous injection of equal volume of solvent two hrs before surgery.

IL-22 monomer 40 µg/kg group (n=7), received a single intravenous injection of 40 µg/kg recombinant human IL-22 (rhIL-22) two hrs before surgery.

IL-22 dimer 100 µg/kg group (n=7), received a single intravenous injection of 100 µg/kg IL-22 dimer (comprising an equal molar IL-22 molecule dosage in comparison to IL-22 monomer 40 µg/kg group) two hrs before surgery.

The IL-22 dimer consisted of two monomeric subunits each comprising a sequence shown in SEQ ID NO: 4.

The animals were given free access to water and fasted for 12 hrs before surgery.

### Surgical procedures:

Rats in the model group were anaesthetized with diethyl ether. The abdomen was opened by a midline incision, the duodenum and common bile duct were identified, then the common bile duct was temporarily occluded at the confluence of hepatic hilus hepatic duct using a microvascular clamp. Upon finding a mesenterium avascular area at lateral wall of duodenum, a 0.4 size needle was used to puncture and sideling insert into the bile-pancreatic duct in the mesenterium avascular area, and then pulled out. A polyethylene (PE) 10 tube was then inserted into the bile-pancreatic duct along the duodenal papilla for 8-10 mm via the hole, and fixed to avoid dropping out. 3.5% sodium taurocholate (0.1 mL/100 g) was slowly infused in a retrograde way, and the needle core was kept staying for 8 mins after injection. Upon removing the polyethylene tube and microvascular clamp, the abdomen was closed. Rats were given free access to food and water after surgery. At 12 hrs after surgery, blood samples were taken from rat orbital venous plexus, and then the serum was separated by centrifuging. The serum levels of amylase and lipase were measured.

The animals were sacrificed 48 hrs after surgery. The pancreas tissues of rats were taken and fixed in 10% formalin solution. Tissues at head, middle, and tail of the pancreas were sliced and made into 3 µm paraffin sections, respectively. The sections were stained with HE, and the pathological changes were observed under a light microscope. Scores of edema, necrosis, hemorrhage, inflammatory cell infiltration, etc. were evaluated in a double blind fashion, according to the scales of Schmidt (Schmidt et al. Ann Surg, 1992, 215(1):44-56). Scoring of 3 sections including the head, middle, and tail of the pancreas for each rat was performed.

### Results:

The pancreatitis animal model was successfully established, as evidenced by a significant elevation in serum levels of amylase and lipase. As shown in FIG.9A and 9B, compared to the model group, IL-22 monomer has a trend to decrease the serum levels of amylase, but there was no significant difference. The serum levels of amylase were significantly decreased after the IL-22 dimer treatment (P=0.03). Compared to the model group, the serum levels of lipase were significantly decreased (P=0.03) after the IL-22 monomer treatment, whereas the serum levels of lipase were significantly decreased after the IL-22 dimer treatment (P=0.008). It is worth noting that, at equal molar IL-22 dosage, the IL-22 dimer was therapeutically effective in pancreatitis rat model, and the efficacy was better than that of IL-22. Under a microscope, obvious edema, a mass of inflammatory cell infiltration, necrosis of partial acinar cell and adipose cell, and a small amount of hemorrhage were observed in the pancreatic tissues of model group. IL-22 dimer can significantly improve the pathology score in animals of pancreatitis, showing a protective role on pancreas. At equal molar IL-22 dosage, no significant protective effect of IL-22 monomer on pancreas was observed.

**Table 5 The pathology scores of pancreatic tissue in rats**

| | Edema | Inflammatory cell infiltration | Necrosis of acinar cell | Hemorrhage | Necrosis of adipose cell | Total |
|---|---|---|---|---|---|---|
| Model group | 6.2 ± 1.8 | 7.0 ± 1.2 | 3.8 ± 2.2 | 2.4 ± 2.1 | 1.4 ± 0.9 | 20.8 ± 4.0 |
| IL-22 monomer group 40 µg/kg | 7.4 ± 1.7 | 5.7 ± 1.6 | 2.4 ± 1.7 | 3.7 ± 3.4 | 0.9 ± 0.7 | 20.1 ± 4.0 |
| IL-22 dimer group 100 µg/kg | 4.3 ± 2.7^{b} | 5.7 + 2.3 | 2.3 ± 0.5 | 2.3 ± 2.1 | 0.5 ± 0.8 | 15.2 ± 3.8 |

| | | | | | | |
|---|---|---|---|---|---|---|
| a indicating P<0.05 compared to the model group. b indicating P<0.05 compared to the IL-22 monomer group | | | | | | |

## Claims

1. An IL-22 dimer for use in a method of treating pancreatitis in an individual, wherein the method comprises administering to the individual an effective amount of the IL-22 dimer, wherein the IL-22 dimer is administered at the effective amount of 2 µg/kg to 200 µg/kg.

2. The IL-22 dimer for use according to claim 1, wherein the IL-22 dimer comprises two monomeric subunits, wherein each monomeric subunit comprises an IL-22 domain and a dimerization domain, optionally wherein the monomeric subunit comprises an IL-22 domain linked to the dimerization domain via an optional linker sequence.

3. The IL-22 dimer for use according to claim 2, wherein the linker sequence is about 5 to about 50 amino acids, optionally wherein the linker sequence comprises the sequence of SEQ ID NO: 1 or SEQ ID NO: 10, for example wherein the linker sequence consists of the sequence of SEQ ID NO:1 or SEQ ID NO: 10.

4. The IL-22 dimer for use according to any one of claims 1-3, wherein the dimerization domain comprises at least two cysteines capable of forming intermolecular disulfide bonds.

5. The IL-22 dimer for use according to any one of claims 1-4, wherein the dimerization domain comprises at least a portion of the Fc fragment, optionally wherein the Fc fragment comprises CH2 and CH3 domains, and/or wherein the Fc fragment comprises the sequence of SEQ ID NO:2 or SEQ ID NO:9, for example wherein the Fc fragment consists of the sequence of SEQ ID NO:2 or SEQ ID NO:9.

6. The IL-22 dimer for use according to any one of claims 2-5, wherein the IL-22 domain of each of the monomeric subunits has the sequence of SEQ ID NO:3.

7. The IL-22 dimer for use according to any one of claims 2-6, wherein each of the monomeric subunits comprises an amino acid sequence selected from SEQ ID NO:4 and SEQ ID NOs:6-8.

8. The IL-22 dimer for use according to any one of claims 1-7, wherein the IL-22 dimer is administered intravenously.

9. The IL-22 dimer for use according to any one of claims 1-8, wherein the IL-22 dimer is administered at the amount of:
(i) 5 µg/kg to 80 µg/kg; or
(ii) 10 µg/kg to 45 µg/kg.

10. The IL-22 dimer for use according to any one of claims 1-9, wherein the pancreatitis is:
(i) acute pancreatitis;
(ii) chronic pancreatitis;
(iii) alcohol induced pancreatitis;
(iv) recurrent pancreatitis;
(v) bile reflux pancreatitis;
(vi) interstitial pancreatitis;
(vii) necrotizing pancreatitis; or
(viii) post ERCP pancreatitis.

11. The IL-22 dimer for use according to any one of claims 1-10, wherein the individual has elevated serum amylase, elevated serum lipase, elevated urine amylase, or elevated C-reactive protein.

12. The IL-22 dimer for use according to any one of claims 1-11, wherein the IL-22 dimer is administered:
(i) no more than about once a week;
(ii) no more than about once a month; or
(iii) no more than about once every three months.

13. The IL-22 dimer for use according to any one of claims 1-12, wherein the effective amount of the IL- 22 dimer:
(i) leads to an increased level of serum amyloid A protein or C-reactive protein in the individual;
(ii) leads to inhibition of triglyceride in the individual; or
(iii) does not lead to an elevated level of an inflammatory cytokine, optionally wherein the inflammatory cytokine is selected from the group consisting of TNFα, IL-6, IL-1β, and IL-8.

14. The IL-22 dimer for use according to any one of claims 2-13, wherein the dimerization domain is:
(i) at the N-terminus of each IL-22 monomeric subunit; or
(ii) at the C-terminus of each IL-22 monomeric subunit.

## Patentansprüche

1. IL-22-Dimer zur Verwendung bei einem Verfahren zur Behandlung von Pankreatitis bei einem Individuum, wobei das Verfahren das Verabreichen einer wirksamen Menge des IL-22-Dimers an das Individuum umfasst, wobei das IL-22-Dimer mit der wirksamen Menge von 2 µg/kg bis 200 µg/kg verabreicht wird.

2. IL-22-Dimer zur Verwendung nach Anspruch 1, wobei das IL-22-Dimer zwei monomere Untereinheiten umfasst, wobei jede monomere Untereinheit eine IL-22-Domäne und eine Dimerisierungsdomäne umfasst, wobei die monomere Untereinheit gegebenenfalls eine IL-22-Domäne umfasst, die über eine optionale Linkersequenz an der Dimerisierungsdomäne gebunden ist.

3. IL-22-Dimer zur Verwendung nach Anspruch 2, wobei die Linkersequenz etwa 5 bis etwa 50 Aminosäuren aufweist, wobei die Linkersequenz gegebenenfalls die Sequenz SeQ ID Nr.: 1 oder SEQ ID Nr.: 10 umfasst, wobei die Linkersequenz beispielsweise aus der Sequenz SeQ ID Nr.: 1 oder SEQ ID Nr.: 10 besteht.

4. IL-22-Dimer zur Verwendung nach einem der Ansprüche 1-3, wobei die Dimerisierungsdomäne wenigstens zwei Cysteine umfasst, die zur Bildung von intermolekularen Disulfidbindungen fähig sind.

5. IL-22-Dimer zur Verwendung nach einem der Ansprüche 1-4, wobei die Dimerisierungsdomäne wenigstens einen Teil des Fc-Fragments umfasst, wobei das Fc-Fragment gegebenenfalls CH2- und CH3-Domänen umfasst und/oder wobei das Fc-Fragment die Sequenz SEQ ID Nr.: 2 oder SEQ ID Nr.: 9 umfasst, wobei das Fc-Fragment beispielsweise aus der Sequenz SEQ ID Nr.: 2 oder SEQ ID Nr.: 9 besteht.

6. IL-22-Dimer zur Verwendung nach einem der Ansprüche 2-5, wobei die IL-22-Domäne einer jeden der monomeren Untereinheiten die Sequenz SEQ ID Nr.: 3 aufweist.

7. IL-22-Dimer zur Verwendung nach einem der Ansprüche 2-6, wobei jede der monomeren Untereinheiten eine Aminosäuresequenz umfasst, die aus SEQ ID Nr.: 4 und SEQ ID Nr.: 6-8 ausgewählt ist.

8. IL-22-Dimer zur Verwendung nach einem der Ansprüche 1-7, wobei das IL-22-Dimer intravenös verabreicht wird.

9. IL-22-Dimer zur Verwendung nach einem der Ansprüche 1-8, wobei das IL-22-Dimer in der Menge von:
(i) 5 µg/kg bis 80 µg/kg oder
(ii) 10 µg/kg bis 45 µg/kg
verabreicht wird.

10. IL-22-Dimer zur Verwendung nach einem der Ansprüche 1-9, wobei es sich bei der Pankreatitis um Folgendes handelt:
(i) akute Pankreatitis;
(ii) chronische Pankreatitis;
(iii) alkoholinduzierte Pankreatitis;
(iv) rezidivierende Pankreatitis;
(v) Gallenreflux-Pankreatitis;
(vi) interstitielle Pankreatitis;
(vii) nekrotisierende Pankreatitis oder
(viii) Pankreatitis nach ERCP.

11. IL-22-Dimer nach einem der Ansprüche 1-10, wobei das Individuum erhöhte Amylase im Serum, erhöhte Lipase im Serum, erhöhte Amylase im Urin oder erhöhtes C-reaktives Protein aufweist.

12. IL-22-Dimer zur Verwendung nach einem der Ansprüche 1-11, wobei das IL-22-Dimer:
(i) nicht mehr als etwa einmal pro Woche;
(ii) nicht mehr als etwa einmal pro Monat oder
(iii) nicht mehr als etwa einmal alle drei Monate
verabreicht wird.

13. IL-22-Dimer zur Verwendung nach einem der Ansprüche 1-12, wobei die wirksame Menge des IL-22-Dimers:
(i) zu einem erhöhten Spiegel von Serum-Amyloid-A-Protein oder C-reaktivem Protein im Individuum führt;
(ii) zu einer Hemmung von Triglycerid im Individuum führt oder
(iii) nicht zu einem erhöhten Spiegel eines inflammatorischen Zytokins führt, wobei das inflammatorische Zytokin gegebenenfalls ausgewählt ist aus der Gruppe bestehend aus TNFα, IL-6, IL-1β und IL-8.

14. IL-22-Dimer zur Verwendung nach einem der Ansprüche 2-13, wobei sich die Dimerisierungsdomäne:
(i) am N-Terminus einer jeden monomeren IL-22-Untereinheit oder
(ii) am C-Terminus einer jeden monomeren IL-22-Untereinheit
befindet.

## Revendications

1. Dimère d'IL-22 destiné à être utilisé dans un procédé de traitement d'une pancréatite chez un individu, dans lequel le procédé comprend une administration à l'individu d'une quantité efficace du dimère d'IL-22, le dimère d'IL-22 étant administré en la quantité efficace de 2 µg/kg jusqu'à 200 µg/kg.

2. Dimère d'IL-22 destiné à être utilisé selon la revendication 1, le dimère d'IL-22 comprenant deux sous-unités monomériques, dans lequel chaque sous-unité monomérique comprend un domaine d'IL-22 et un domaine de dimérisation, en option dans lequel la sous-unité monomérique comprend un domaine d'IL-22 lié au domaine de dimérisation via une séquence lieuse optionnelle.

3. Dimère d'IL-22 destiné à être utilisé selon la revendication 2, dans lequel la séquence lieuse est de 5 acides aminés environ jusqu'à 50 environ, en option dans lequel la séquence lieuse comprend la séquence de SEQ ID n° : 1 ou de SEQ ID n° : 10, par exemple dans lequel la séquence lieuse est constituée par la séquence de SEQ ID n° : 1 ou de SEQ ID n° : 10.

4. Dimère d'IL-22 destiné à être utilisé selon l'une quelconque des revendications 1 à 3, dans lequel le domaine de dimérisation comprend au moins deux cystéines capables de former des liaisons disulfure intermoléculaires.

5. Dimère d'IL-22 destiné à être utilisé selon l'une quelconque des revendications 1 à 4, dans lequel le domaine de dimérisation comprend au moins une partie du fragment Fc, en option dans lequel le fragment Fc comprend des domaines CH2 et CH3, et/ou dans lequel le fragment Fc comprend la séquence de SEQ ID n° : 2 ou de SEQ ID n° : 9, par exemple dans lequel le fragment Fc est constitué par la séquence de SEQ ID n° : 2 ou de SEQ ID n° : 9.

6. Dimère d'IL-22 destiné à être utilisé selon l'une quelconque des revendications 2 à 5, dans lequel le domaine d'IL-22 de chacune des sous-unités monomériques a la séquence de SEQ ID n° : 3.

7. Dimère d'IL-22 destiné à être utilisé selon l'une quelconque des revendications 2 à 6, dans lequel chacune des sous-unités monomériques comprend une séquence d'acides aminés choisie parmi les SEQ ID n° : 4 et SEQ ID n° : 6 à 8.

8. Dimère d'IL-22 destiné à être utilisé selon l'une quelconque des revendications 1 à 7, le dimère d'IL-22 étant administré par voie intraveineuse.

9. Dimère d'IL-22 destiné à être utilisé selon l'une quelconque des revendications 1 à 8, le dimère d'IL-22 étant administré en la quantité de :
(i) de 5 µg/kg jusqu'à 80 µg/kg ; ou
(ii) de 10 µg/kg jusqu'à 45 µg/kg.

10. Dimère d'IL-22 destiné à être utilisé selon l'une quelconque des revendications 1 à 9, dans lequel la pancréatite est :
(i) une pancréatite aiguë ;
(ii) une pancréatite chronique ;
(iii) une pancréatite induite par l'alcool ;
(iv) une pancréatite récurrente ;
(v) une pancréatite par reflux biliaire ;
(vi) une pancréatite interstitielle ;
(vii) une pancréatite nécrosante ; ou
(viii) une pancréatite post-ERCP.

11. Dimère d'IL-22 destiné à être utilisé selon l'une quelconque des revendications 1 à 10, dans lequel l'individu a une amylase sérique élevée, une lipase sérique élevée, une amylase urinaire élevée ou une protéine C-réactive élevée.

12. Dimère d'IL-22 destiné à être utilisé selon l'une quelconque des revendications 1 à 11, le dimère d'IL-22 étant administré :
(i) au maximum une fois par semaine environ ;
(ii) au maximum une fois par mois environ ; ou
(iii) au maximum une fois tous les trois mois environ.

13. Dimère d'IL-22 destiné à être utilisé selon l'une quelconque des revendications 1 à 12, dans lequel la quantité efficace du dimère d'IL-22 :
(i) conduit à un taux accru de protéine amyloïde A sérique ou de protéine C-réactive chez l'individu ;
(ii) conduit à une inhibition des triglycérides chez l'individu ; ou
(iii) ne conduit pas à un taux élevé d'une cytokine inflammatoire, en option dans lequel la cytokine inflammatoire est choisie dans le groupe constitué par le TNFα, l'IL-6, l'IL-1β et l'IL-8.

14. Dimère d'IL-22 destiné à être utilisé selon l'une quelconque des revendications 2 à 13, dans lequel le domaine de dimérisation est :
(i) à l'extrémité N-terminale de chaque sous-unité monomérique d'IL-22 ; ou
(ii) à l'extrémité C-terminale de chaque sous-unité monomérique d'IL-22.
